# EUROPEAN PATENT SPECIFICATION

(11) **EP 0 233 045 B1**
(45) Date of publication and mention of the grant of the patent: **24.05.1995**
(21) Application number: 87300961.7
(22) Date of filing: 03.02.1987
(51) Int. Cl.: C12N 15/48, C07K 14/16, G01N 33/569, C12N 5/10, C12P 21/02, A61K 39/21

(54) **Peptides for the diagnose of HTLV-III antibodies, their preparation and use**
Peptide zur Diagnose von HTLV-III-Antikörpern, deren Herstellung und Verwendung
Peptides pour le diagnostic des anticorps HTLV-III, ainsi que leur préparation et leur utilisation

(30) Priority: 03.02.1986 US 825597; 25.09.1986 US 911455
(43) Date of publication of application: 19.08.1987
(73) Proprietor: CAMBRIDGE BIOTECH CORPORATION, Worcester Massachusetts 01605 (US)
(72) Inventor: Thorn, Richard M., Milford Massachusetts 01757 (US); Marciani, Dante Juan, Hopkinton Massachusetts 01748 (US); Hung, Chung-Ho, Milford Massachusetts 01757 (US); Beltz, Gerald A., Lexington Massachusetts 02173 (US); Haseltine, William A., Cambridge, Massachusetts 02115 (US)
(74) Representative: Sheard, Andrew Gregory

(56) References cited:
- EP-A- 0 138 667
- EP-A- 0 181 150
- EP-A- 0 187 041
- EP-A- 0 199 301
- EP-A- 0 199 438
- EP-A- 0 201 716
- EP-A- 0 206 842
- WO-A-85/04903
- WO-A-86/02383
- WO-A-86/02930
- WO-A-86/06414
- GB-A- 2 181 435
- SCIENCE, vol. 228, April 5, 1985, pp. 93-96; N.T. CHANG et al.
- CELL, vol. 41, May 1985, pp. 979-986; R. CROWL et al.
- BIO/TECHNOLOGY, vol. 4, February 1986, pp. 128-133; C.D. CABRADILLA et al.
- NATURE, vol. 313, February 7, 1985, pp. 450-458; MUESING et al.
- SCIENCE, vol. 227, February 1, 1985, pp. 484-492; R. SANCHEZ-PESCADOR et al.
- DNA, vol. 5, no. 2, February 1986, pp. 93-99; J. GHRAYEB et al.
- PROCEEDING OF THE NATIONAL ACADEMY OF SCIENCES OF THE USA, vol. 82, no. 22, November 1985, pp. 7748-7752; Washington, US, D.H. DOWBENKO et al.
- NATURE, vol. 315, May 9, 1985, pp. 151-154; New York, US, N.T. CHANG et al.
- PROCEEDINGS OF THE NATIONAL ACADEMY OF SCIENCES OF THE USA, vol. 82, August 1985, pp. 5199-5202; F. DI MARZO VERONESE et al.
- SCIENCE, vol. 225, July 20, 1984, pp. 321-323, V.S. KALYANARAMAN et al.
- JOURNAL OF EXPERIMENTAL MEDICINE, vol. 159, April 1984, pp. 1117-1131; T.J. PALKER et al.

## Description

### Field of the Invention

This invention is directed to the discovery that certain peptide fragments of the human T-cell leukemia (lymphotropic) virus (HTLV-III) are particularly immunoreactive to HTLV-III antibodies. These fragments can therefore be applied to immunodiagnostic tests for the detection of antibodies to HTLV-III.

### Background of the Invention

The human T-cell leukemia (lymphotropic) virus (HTLV-III) is a retrovirus which carries its genetic code on RNA. When a retrovirus infects a host cell, a DNA copy of its genome is integrated into the chromosome of its host. With some retroviruses, the DNA is integrated into the host cell's chromosomes in the form of a sequence known as a provirus. The DNA copy of the retrovirus' genetic code is synthesized by a viral enzyme called RNA dependent DNA polymerase, or reverse transcriptase. The host cells transcribe the DNA of the viral gene and synthesize the proteins encoded by the virus, which are then assembled into new viruses.

The HTLV-III RNA genome is similar to those of other retroviruses and contains at least (i) a gag gene that encodes the internal structural (nucleocapsid or core) proteins, (ii) a pol gene that encodes the reverse transcriptase, and (iii) an env gene that encodes the envelope glycoproteins of the virus. HTLV-III contains additional genes including those designated tat, sor, and 3'-ORF.

The complete DNA nucleotide sequence for HTLV-III has been reported in the literature by several researchers: Ratner et al., Nature, 313:277-284 (1985); Muesing et al., Nature, 313:450-485 (1985); Sanchez-Pescador et al, Science, 227:44-492 (1985); and Wain-Hopsin et al, Cell, 40:9-17 (1985).

Others have shown that the entire HTLV-III envelope protein can be used for diagnosis of the HTLV-III virus. Allan et al., Science, 228:1091-1094 (1985); Barin et al., Science, 228: 1094-1096 (1985); and Veronese et al., Science, 229:1402-1405 (1985).

Molecular cloning of portions of the virus sequence has been achieved, as described in Ratner et al., supra; Chang et al., Bio/Technology, 3:905-909 (1985); Chang et al., Science, 228:93-96 (1985); and Crowl et al., Cell, 41:979-986 (1985). These clones provide material for analysis of possible polypeptide epitopes on the HTLV-III viral surface against which the immune system can mount an antibody reaction. Identification of these epitopes is important in the development of sensitive and rapid methods for the diagnosis of AIDS.

### Summary of the Invention

Recognizing the role that epitopes on the viral surface play in immunological diagnosis, the inventors evaluated the provirus of the human T-cell leukemia virus-type III (HTLV-III) in an effort to identify diagnostic regions. These efforts have culminated in the identification of specific peptide fragments or constructs thereof which are particularly immunoreactive to anti-HTLV-III antibodies. The inventors have further modified these diagnostic peptides, increasing significantly the degree of immunoreactivity. The invention provides a peptide fragment which is immunoreactive to antibodies against the HTLV-III virus and encoded by the HTLV-III provirus nucleotide sequence 7199 to 8052.

The inventors then successfully achieved high levels of expression of this diagnostic peptide by genetic engineering methods.

Diagnostic assays involving the use of this peptide have also been developed for the detection of antibodies to HTLV-III in samples suspected of containing antibodies against the HTLV-III virus.

### Brief Description of the Drawings

Figure 1 shows the preparation of the plasmids pJLB0, pJLB1, and pJLB2 from pJLA16.

Figure 2 shows the construction of the addition of translation polyterminator to the plasmids of Figure 1, and the resulting plasmids pJLB0T, pJLB1T, and pJLB2T.

Figure 3 shows the removal of the repressor of transcription required for priming of DNA synthesis from the plasmids of Figure 2, and the resulting plasmids pJLB0TR, pJLB1TR, and pJLB2TR.

Figure 4 shows the construction of intermediate plasmids delta pBR2 and delta pBR4 from pBR322.

Figure 5 shows the construction of the subcloning of the lambda pL fragment into the plasmid delta pBR4.

Figure 6 shows the construction of the subcloning of the lambda cII Shine-Dalgarno sequencing behind the lambda pL promoter resulting in plasmid designated delta pBR4-pL-cII.

Figure 7 shows the two step subcloning of the Taq 1 fragments into plasmid delta pBR4-pL-cII, and the resulting plasmid denoted plasmid delta pBR4-pL-cII-Taq I.

Figure 8 shows the construction of the addition of translation polyterminator to plasmid delta pBR4-pL-cII-Taq I, and the resulting plasmid delta pBRPT-pL-cII-OSD.

Figure 9 shows the construction of the plasmid delta pBRPT-pL-cII-NSD from the plasmid of Figure 8.

Figure 10 shows the construction of the plasmids pLCBCO, pLCBC1, and pLCBC2 from the plasmid delta pBR4PT-pL-cII-NSD and the construction of the plasmids pLCBC00-pLCB10, and pLCBC20 from the plasmid delta pBR4PT-pL-cII-OSD.

Figure 11 shows the random cloning strategy for expressing clones.

Figure 12 shows the direct cloning strategy for expressing clones.

Figure 13 shows the restriction fragments obtained from the direct cloning strategy.

Figure 14 shows the modifications to clone G for increased expression.

Figures 15-19 show five particularly immunodiagnostic regions of HTLV-III. The nucleotide sequences are respectively, Figure 15: 653-1218; Figure 16: 2600-3911; Figure 17: 2743-4211; Figure 18: 6619-7198; and Figure 19: 7199-8052. Legend: BH10 and BH5 represent clone nomenclature; the set of numbers to the left of the sequences represent nucleotide residues.

Figure 20 shows the ELISA assay using the peptide fragment delta G-71A on both control and human patient sera.

Figure 21 shows the DNA sequence of the clone delta G-71A and the corresponding amino acid sequence which is coded therefor.

### Definitions

In the description that follows, a number of terms used in recombinant DNA technology are extensively utilized. In order to provide a clearer and consistent understanding of the specification and claims, including the scope to be given such terms, the following definitions are provided.

Promoter. A DNA sequence generally described as the 5' region of a gene, located proximal to the start codon. At the promoter region, transcription of an adjacent gene(s) is initiated.

Gene. A DNA sequence that contains information for construction of a polypeptide or protein, and as used herein, includes the 5' and 3' ends.

Structural gene. A DNA sequence that is transcribed into messenger RNA that is then translated into a sequence of amino acids characteristic of a specific polypeptide. Typically the first nucleotide of the first translated codon is numbered +1, and the nucleotides are numbered consecutively with positive integers through the translated region of the structural gene and into the 3' untranslated region. The numbering of nucleotides in the promoter and regulatory region 5' to the translated region proceeds consecutively with negative integers with the 5' nucleotide next to the first translated nucleotide being numbered -1.

Operably linked. As used herein means that the promoter controls the initiation of the expression of the polypeptide encoded by the structural gene.

Expression. Expression is the process by which a structural gene produces a polypeptide. It involves transcription of the gene into messenger RNA (mRNA) and the translation of such mRNA into polypeptide(s).

Cloning vehicle. A plasmid or phage DNA or other DNA sequence which is able to replicate in a host cell, which is characterized by one or a limited number of endonuclease recognition sites at which such DNA sequences may be cut in a determinable fashion without loss of an essential biological function of the DNA, and which contain a phenotypic selection marker suitable for use in the identification of transformed cells. Markers, for example, are tetracycline resistance or ampicillin resistance. The word "vector" is sometimes used for cloning vehicle.

Expression vehicle. A vehicle similar to a cloning vehicle but which is capable of expressing a given structural gene in a host, normally under control of certain regulatory sequences. Such vehicles are designed to promote the expression of gene inserts. Typically, a restriction fragment carrying the regulatory sequences of a gene is ligated in vitro to a plasmid containing a restriction fragment possessing the gene but lacking its regulatory sequences. The plasmid with this new combination of DNA sequences is then cloned under circumstances that promote the expression of the gene under the control of the regulatory sequences.

Host. Any organism that is the recipient of a replicable expression vehicle.

### Description of the Preferred Embodiments

The complete DNA nucleotide sequence and the predicted amino acid sequence for the HTLV-III provirus from the H9/HTLV-III cell line is described in Ratner, et al., Nature, 313: 277-284 (1985). Clones BH5 and BH8, isolated from cell line H9, constituted the starting material of the invention herein described. The H9/HTLV-III cell line is deposited in the American Type Culture Collection under ATCC No. CRL 8543 and generally described in U.S. Patent 4,520,113. The HTLV-III virus can also be propagated using lysates of immortalized human T-cell clones.

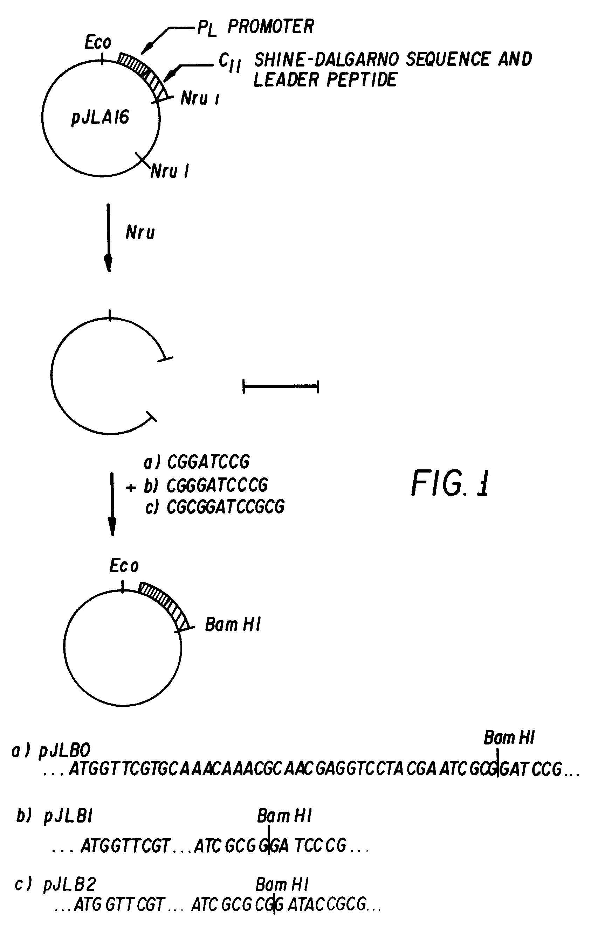

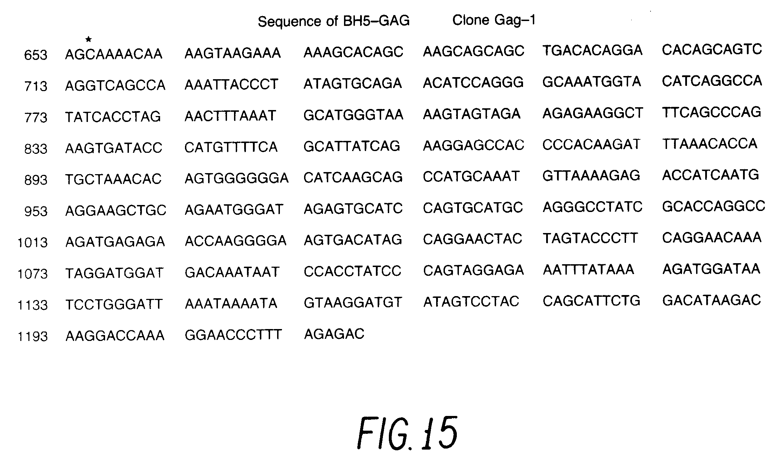

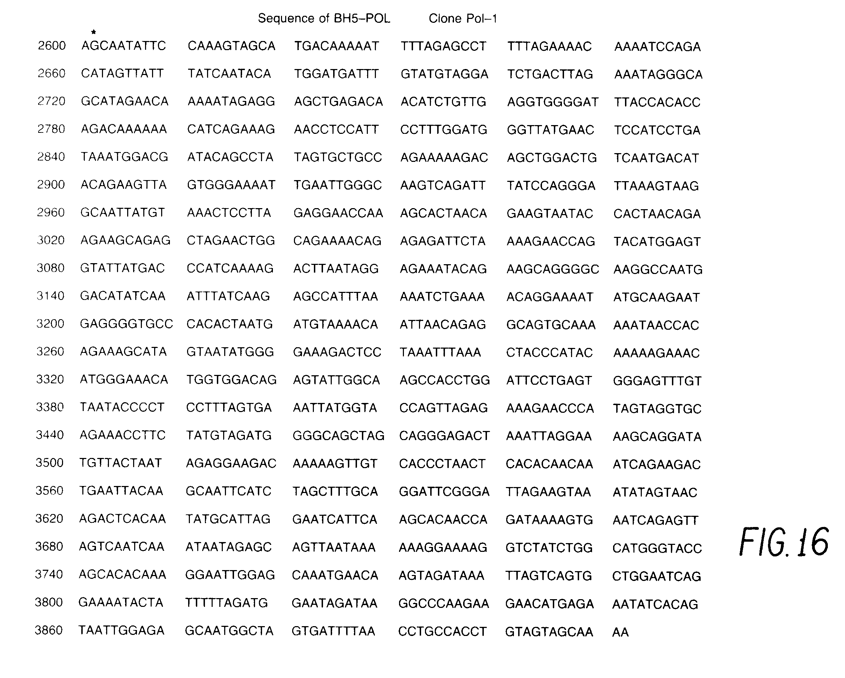

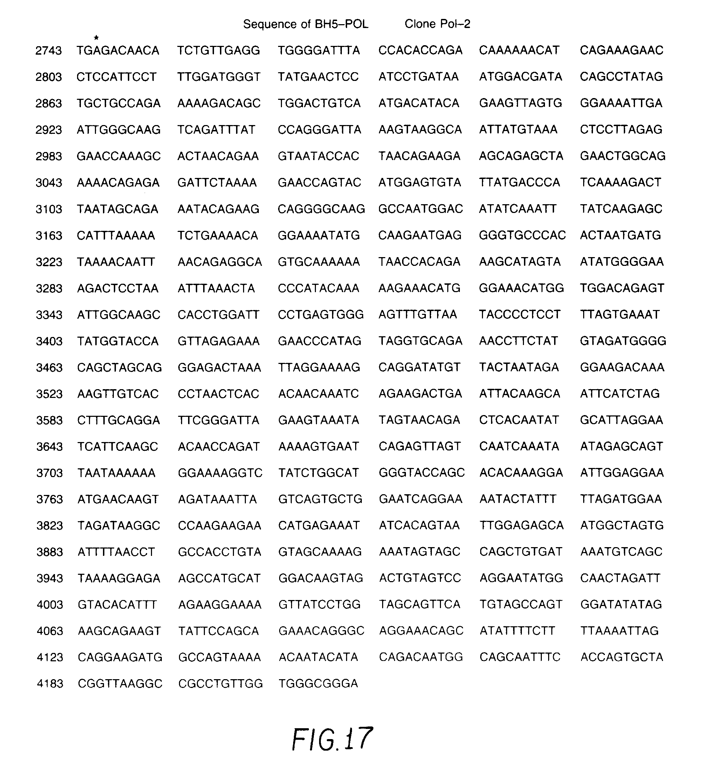

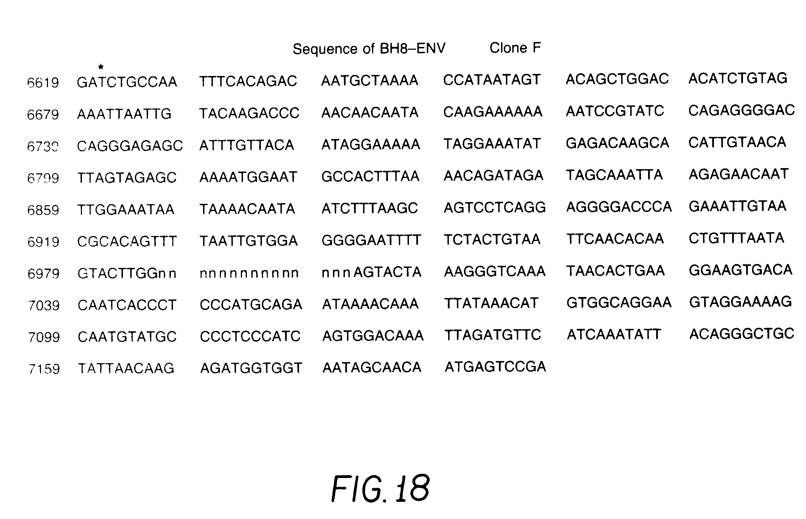

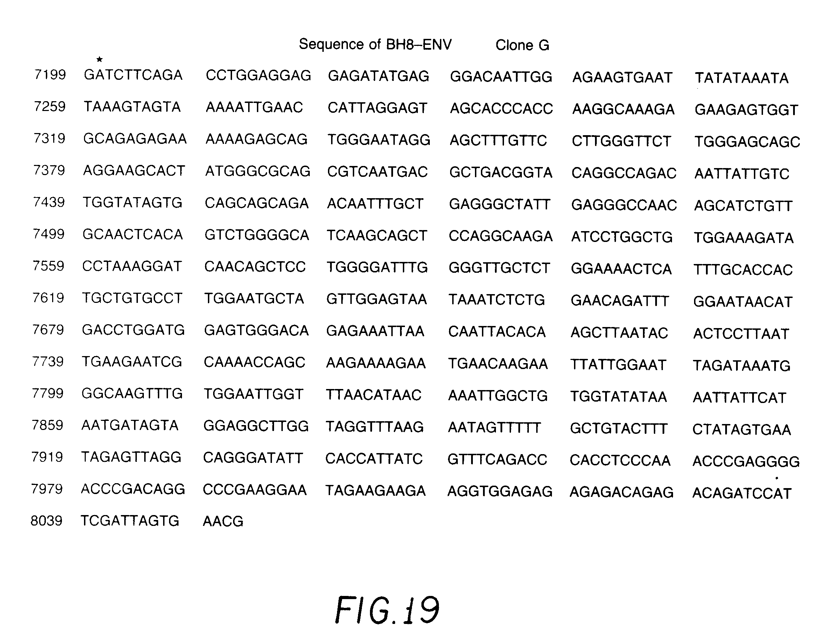

Various nucleotide sequences encoding peptide fragments of the BH5 and BH8 clones were evaluated to determine immunodiagnostic regions. The particular peptide fragments of the HTLV-III provirus discovered by the inventors to be particularly immunoreactive, and therefore immunodiagnostic for AIDS antibodies, are encoded for (or present within) the nucleotide sequences 653-1218, 2600-3911, 2743-4211, 6619-7198, and 7199-8052 as that sequence is numbered in Figure 1 of Ratner et al., supra. These five regions are shown in Figures 15-19 of the present application. As used herein and where appropriate, the nucleotide sequence numbering is based on the first nucleotide beginning on the 3' side of the restriction endonuclease clip site.

The peptide fragment of the present invention is (or is within) 7199-8052.

Additionally, the inventors have discovered that deleting the DNA sequence encoding certain hydrophobic region(s) of the fragments results in increased expression of the fragment, and also in a high degree of immunoreactivity to HTLV-III antibodies. The hydrophobic regions are those regions comprising hydrophobic amino acids encoded for by various nucleotide segments of the HTLV-III virus. In a preferred embodiment, two hydrophobic regions of the immunogenic peptide fragment encoded for by the nucleotide sequences 7199-8052 have been deleted. These two hydrophobic regions are encoded approximately between nucleotides 7352 and 7418 and approximately betwen nucleotides 7851 and 7916.

Moreover, the inventors have also discovered that extending the amino end of a peptide fragment having the hydrophobic region deleted results in increased expression of the fragment and in a high degree of immunoreactivity to HTLV-III antibodies. Thus, in another preferred embodiment, the amino end of the hydrophobic double-deleted peptide fragment encoded for by nucleotide sequences 7199-8052 is extended. In the most preferred embodiment, the amino end of the hydrophobic double-deleted peptide fragment encoded for by nucleotide sequences 7199-8052 is extended to the Aha III site, at about nucleotide 6827. This portion of the HTLV-III, nucleotides 6827 to 8052, contains nearly all of the conserved envelope sequences among HTLV-III isolates known to date. This particular peptide fragment has been designated delta G-71A and an E. coli culture (MZl) containing a plasmid that expresses this peptide has been designated p-delta-G71AC. This culture was deposited in American Type Culture Collection, 12301 Parklawn Dr., Rockville, Maryland 20852, under ATCC No. 53455 on January 30, 1986. This depository assures permanence of the deposit and ready accessibility thereto by the public.

As will be understood by one of skill in the art, there may be variations in the first one or two amino acids of the peptide fragments due to proper alignment of the cloned nucleotide sequence in the expression vehicle.

Further, the peptide fragments may be subject to various changes, such as insertions, deletions and substitutions, either conservative or non-conservative, where such changes may enhance the immunoreactivity of the peptide to the anti-HTLV-III antibodies. By conservative substitutions is intended combinations such as gly, ala; val, ile, leu; asp, glu; asn, gln; ser, thr; lys, arg; and phe, tyr.

The nucleotide sequence of mRNA is translated in groups of three nucleotides, called codons. Each codon is represented by a single amino acid in the peptide fragment synthesized. The reading-frame defines which sets of three nucleotides are read as codons, determined by the initiation codon AUG.

Of particular interest are peptides of the following formula:
1) H₂N--X--CO--R¹
   wherein R¹ is Cys-CO-R², OH, OM or -NR³R⁴;
   M is a pharmaceutically acceptable cation or a lower (C₁-C₆) branched or unbranched alkyl group;
   R², R³ and R⁴ are the same or different and selected from the group consisting of hydrogen and a lower (C₁-C₆) branched or unbranched alkyl group; and
   X is the amino acid sequence or peptide fragment as described above;
2) the acid addition salts thereof; and
3) the protected or partially protected derivatives thereof.

As is known in the art, the amino acid residues may be in their protected or unprotected form, using appropriate amino or carboxyl protecting groups.

Useful cations M are alkali or alkaline earth metallic cations (i.e., Na, K, Li, 1/2 Ca, 1/2 Ba, etc.) or amine cations (i.e., tetraalkylammonium, trialkylammonium, where alkyl can be C₁-C₁₂).

The variable length peptides may be in the form of the free amines (on the N-terminus), or acid-addition salts thereof. Common acid addition salts are hydrohalic acid salts, i.e., HBr, HI, or more preferably, HCl.

The peptide fragments of the HTLV-III virus may be obtained from the provirus in a host cell. The peptide fragment would then be obtained by fragmenting the naturally-occurring virus using suitable enzymes or chemical methods. However, it is possible to obtain the peptide fragment by synthesis, for example, by well known solid phase peptide synthesis described in Merrifield, J. Am. Chem. Soc., 85: 2149 (1962) and Stewart and Young in Solid Phase Peptide Synthesis (Freeman, San Francisco 1969) at 27-62.

A preferred method of obtaining the peptide fragment is by cloning the desired polynucleotide fragment utilizing genetic engineering techniques. The advantages of using genetic engineering and recombinant clones are twofold: the first advantage is that it is difficult and time-consuming to obtain large amounts of the viral genome either by isolation techniques or by synthesis; the second advantage is that recombinant peptides are devoid of human antigens that may reduce the reliability of a diagnostic test.

The term "peptide fragment," is thus meant to include naturally-occurring amino acid sequences, synthetic sequences, and expressed fragments from cloned sequences representing segments of the HTLV-III viral genome.

It will be understood by one of skill in the art that there may be some variation in the diagnostic peptide fragments, as described above, provided however, that these peptides retain immunoreactivity to antibodies to the HTLV-III virus. Thus, the ranges in the length of the peptide fragments need not be precisely fixed. Amino acids of the peptide fragments may be deleted or added without loss of immunoreactivity. Additionally, amino acids could be exchanged, e.g. a neutral amino acid such a valine could be exchanged with another neutral amino acid, such as leucine. There is also genomic variation from isolate to isolate. (See, for example, Wong-Staal et al., Science, 229:759-762 (1985)). It is to be understood that such variations are included in the peptide fragments of this invention.

The genetic constructs and the methods for using them can be utilized for expression of the peptide fragments in hosts, including procaryotic and eucaryotic hosts. The procaryotic hosts may include bacteria, such as E. coli and S. typhimurium, Serratia marcescens, or Bacillus subtilis. The preferred bacterial host for expression is an E. coli strain that contains a temperature sensitive bacteriophage lamda CI857 gene, such as MZl, described in Lautenberger et al., Gene Anal. Tech, 1:63-66 (1984). Eucaryotic hosts may include yeast, filamentous fungi, and mammalian cells. The DNA sequence of the peptide fragments can be inserted into the genome for vaccinia virus. (Mackett, M. et al., Proc. Natl. Acad. Sci. USA, 79:7415 (1982); Panicali, D. et al., Proc. Natl. Acad. Sci. USA, 79:4927 (1982); Panicali, D. et al., Proc. Natl. Acad. Sci. USA, 80:5364 (1983); and Smith, G.L. et al., Nature, 302:490 (1983).) The recombinant vaccinia virus then replicates in any mammalian cell and the fragment of interest appears on the envelope or in internal viral proteins. Insect cells can also be used for replication of the fragments. (See, for example, Smith et al., Molecular and Cell Biology, 3:2156-2165 (1983)).

In general, expression vectors containing promoter sequences which facilitate the efficient transcription of the inserted gene fragment, which are derived from species compatible with the host cells, are used in connection with these hosts. The expression vector typically contains an origin of replication, promoters, terminators, as well as specific genes which are capable of providing phenotypic selection in transformed cells.

The transformed host cell can be fermented and cultured according to means known in the art to achieve optimal cell growth, and also to achieve optimal expression of the cloned HTLV-III peptide fragments. As described hereinbelow in Example 5, high level expression of the cloned HTLV-III virus sequences coding for peptide fragments can be achieved according to a preferred procedure of this invention.

After expression of the cloned HTLV-III peptide fragments, the fragments will typically be recovered and purified according to means known in the art. When bacteria are used as the host, high-level expression of the clones usually results in the formation of insoluble inclusion bodies or aggregates. To purify the expressed proteins, the insoluble inclusion bodies must be made soluble. In the preferred embodiment of this invention, the expressed peptide fragments are purified in a process using derivatization of amino groups by citraconylation, which is more fully described in Example 8.

The purified immunogenic and diagnostic peptide fragments according to this invention are specifically recognized by antibodies produced in response to the HTLV-III virus. The HTLV-III antibodies in blood or tissue samples can be detected using the peptide fragments in immunoassays wherein the peptides can be utilized in liquid phase or bound to a solid phase carrier. In addition, the peptide fragments can be detectably labeled in various ways for use in immunoassays for virus. The preferred immunoassays for detecting HTLV-III antibodies using the peptide fragments of this invention include radioimmunoassays, enzyme-linked immunosorbent assays, (ELISA), or other assays known in the art, such as immunofluorescent assays, chemiluminescent assays, or bioluminescent assays.

Radioactive isotopes which are particularly useful in assays are ³H, ¹²⁵I, ¹³¹I, ³²P, ³⁵S, ¹⁴C, ⁵¹Cr, ³⁶Cl, ⁵⁷Co, ⁵⁸Co, ⁵⁹Fe, ⁷⁵Se, and ¹⁵²Eu.

While radiolabeling represents one embodiment, alternatively, the peptide sequence or antibodies thereto may also be labeled using fluorescent labels, enzyme labels, free radical labels, avidin-biotin labels, or bacteriophage labels, using techniques known to the art (Chard, Laboratory Techniques in Biology, "An Introduction to Radioimmunoassay and Related Techniques," North Holland Publishing Company (1978).

Typical fluorescent labels include fluorescein isothiocyanate, rhodamine, phycoerythrin, phycocyanin, allophycocyanint o-phthaldehyde, and fluorescamine.

Typical chemiluminescent compounds include luminol, isoluminol, aromatic acridinium esters, imidazoles, acridinium salts, and the oxalate esters. Typical bioluminescent compounds include luciferin, luciferase, and aequorin.

Typical enzymes include alkaline phosphatase, beta-galactosidase, glucose-6-phosphate dehydrogenase, maleate dehydrogenase, glucose oxidase, and peroxidase.

Two principal types of enzyme assays are enzyme-linked immunosorbent assay (ELISA) and the homogeneous enzyme immunoassay, also known as enzyme-multiplied immunoassay (EMIT) (Syva Corp.). The EMIT system depends on deactivation of the enzyme in the tracer-antibody complex; the activity can thus be measured without the need for a separation step.

The immunoassays within the scope of the present invention include both immunometric assays and competitive assays.

Immunometric assays include forward sandwich, reverse sandwich immunoassays and simultaneous assay. Each of these terms is well understood by those skilled in the art. The immunometric assays will be described for the detection of antibodies to the HTLV-III. In these assays, the peptide fragment is bound to the solid phase carrier and anti-IgG antibodies are detectably labeled.

In a forward sandwich immunoassay, a sample suspected of containing antibodies against HTLV-III is first incubated with a solid phase immunoabsorbent containing the peptide fragment. Incubation is continued for a period of time sufficient to allow the antibodies in the sample to bind to the immobilized peptide fragment. After the first incubation, the solid phase immunoabsorbent is separated from the incubation mixture and washed to remove interfering substances which also may be present in the sample. Solid phase immunoabsorbent-containing antibodies bound to the immobilized peptide fragments are subsequently incubated for a second time with soluble labeled antibody cross-reactive with a different domain on the antibody to be detected. After the second incubation, another wash is performed to remove unbound labeled antibody from the solid phase immunoabsorbent and to remove non-specifically bound labeled antibody. Labeled antibody bound to the solid phase immunoabsorbent is then detected and the amount of labeled antibody detected serves as a direct measure of the amount of antibodies present in the original sample. Alternatively, labeled antibody which is not associated with the immunoabsorbent complex can also be detected, in which case the measure is in inverse proportion to the amount of antigen present in the sample. Forward sandwich assays are described, for example, in United States Patents 3,867,517; 4,012,294; and 4,376,110.

In a reverse sandwich assay, the sample suspected of containing test antibodies against HTLV-III is initially incubated with labeled anti-antibody, after which the solid phase immunoabsorbent containing immobilized peptide fragment cross-reactive with a different domain on the test antibody is added thereto, and a second incubation is carried out. The initial washing step required by a forward sandwich assay is not required, although a wash is performed after the second incubation. Reverse sandwich assays have been described, for example, in U.S. Patents 4,098,876 and 4,376,110.

In a simultaneous sandwich assay, the sample, the immunoabsorbent having immobilized peptide fragment thereon and labeled soluble antibody specific to a different domain of the test antibody are incubated simultaneously in one incubation step. The simultaneous assay requires only a single incubation and does not require washing steps. The use of a simultaneous assay is a very useful technique, providing ease of handling, homogeneity, reproducibility, linearity of the assays, and high precision. See U.S. Patent 4,376,110 to David et al. .

So-called delayed immunometric assays can also be utilized, as are, for example, described in Chu, U.S. Patent 4,289,747, and Wolters, U.S. Patent 4,343,896.

In each of the above assays, the sample-containing antibody, solid phase immunoabsorbent with immobilized peptide fragment and labeled soluble antibody are incubated under conditions and for a period of time sufficient to allow the test antibodies to bind to the immobilized peptide fragments and to the soluble antibodies. In general, it is desirable to provide incubation conditions sufficient to bind as much antibody as possible, since this maximizes the binding of labeled antibody to the solid phase, thereby increasing the signal. Of course, the specific concentrations of labeled antibodies and immobilized fragments, the temperature and time of incubation, as well as other such assay conditions, can be varied, depending upon various factors including the concentration of antibody in the sample, the nature of the sample, and the like. Those skilled in the art will be able to determine operative and optimal assay conditions for each determination by employing routine experimentation.

There are many solid phase immunoabsorbents which have been employed and which can be used in the present invention. Well known immunoabsorbents include beads formed from glass, polystyrene, paper, polypropylene, dextran, nylon, and other material; tubes formed from or coated with such materials, and the like. The immobilized peptide fragments may be covalently or physically bound to the solid phase immunoabsorbent, by techniques such as covalent bonding via an amide or ester linkage or by absorption. Those skilled in the art will know many other suitable carriers for binding peptide fragments, or will be able to ascertain such, using routine experimentation.

General competitive binding assay techniques useful for the detection of minute amounts of organic molecules such as hormones, proteins, antibodies, and the like are well known in the art. See Chard, supra. Any of these competitive binding assay techniques can be used for the purposes of detecting HTLV-III antibodies. In order to carry out a competitive binding assay, typically a radio-immunoassay (RIA), it is necessary to provide a binding molecule which has affinity for the label-containing antibody raised in response to a peptide fragment, and for the HTLV-III antibody to be tested as well. A small amount of the fluid or tissue sample containing an unknown quantity of HTLV-III antibody is incubated in the presence of the raised labeled antibody and also a known amount of antibody-specific peptide fragment.

An agglutination assay can also be used to detect HTLV-III antibodies. For example, the desired fragment is immobilized on a suitable particle, for example, latex beads, gelatin, red blood cells, nylon, liposomes, gold particles, etc. The presence of antibodies in the sample causes agglutination, similar to that of a precipitation reaction, which can then be detected by such techniques as nephelometry, turbidity, infrared spectrometry, visual inspection, colorimetry, and the like.

The raised antibody is preferably generated with antigenic peptide fragments of the invention. Once the incubation of the test sample with the fragment and tracer-containing antibody is complete, it is necessary to determine the distribution of the tracer-containing molecule between the free and bound (immunocomplexed) form. Usually, but not always, this requires that the bound fraction be physically separated from the free fraction. For example, the specific peptide fragment can be bound to a plate. A variety of other techniques may be used for that purpose, each exploiting physical-chemical differences between the tracer-containing molecule in its free and bound form. The generally available methodologies have been described by Yalow, in Pharmacol. Rev., 28: 161 (1973). These techniques include adsorption, precipitation, salting out techniques, organic solvents, electrophoretic separation, and the like. See Chard, supra, pp. 405-422.

As in the immunometric assays described above, the soluble antibody may be labeled with any detectable label, such as a radiolabel, a fluorescent label, an enzyme label, a free radical label, or a bacteriophage label. Most commonly, the label is a radiolabel or an enzyme label.

The immunogenic peptide fragments according to this invention may be used to stimulate the production of antibodies. In order to stimulate the production of antibody, the peptide fragment may be coupled to a carrier protein such as bovine serum albumin or keyhole limpet hemocyanin (KLH), utilizing techniques well known and commonly used in the art. Preferably, the carrier protein is KLH, linked to the peptide fragment through a cysteine residue.

Additionally, the peptide fragments can be admixed with an immunologically inert or active carrier. Carriers which promote or induce immune responses, such as Freund's complete adjuvant, can be utilized.

The preparation of antisera in animals is a well known technique (see, for example, Chard, supra, pages 385-396). The choice of animal is usually determined by a balance between the facilities available, and the likely requirements in terms of volume of the resultant antiserum. A large species such as goat, donkey and horse may be preferred, because of the larger volumes of serum readily obtained. However, it is also possible to use smaller species such as rabbit or guinea pig which often yield high titer antisera. Usually, subcutaneous injection of the antigenic material (the peptide fragment hapten-carrier protein conjugate) are introduced into the immune system of the animal in which antibodies are to be raised. The detection of appropriate antibodies may be carried out by testing the antisera with appropriately labeled tracer-containing molecules. Fractions that bind tracer-containing molecules are then isolated and further purified if necessary.

Antibodies thus obtained may then be utilized in various immunoassays to identify and quantitate the HTLV-III virus or fragments thereof. Both polyclonal antibodies and monoclonal antibodies, produced by well known techniques as described in Kohler and Milstein, Nature, 256: 496 (1975), raised in response to the peptide fragment of this invention can be utilized in immunoassays.

When one uses immunometric assays to detect the HTLV-III virus or portions thereof, two separate and distinct antibodies are required. One of these antibodies is bound to the solid phase support while the other is detectably labeled. In essence, the two different antibodies, although specific for HTLV-III virus; are cross-reactive with different domains on viral protein. In one embodiment, the two different antibodies may be prepared by using two different peptide fragments according to this invention. The use of antibodies to different peptide fragments, one bound to a carrier and the other detectably labeled, is useful in various sandwich assays.

Alternatively, it is also possible to prepare antibodies which are specific to the HTLV-III virus, but cross-reactive with different domains by producing the antisera in two different species, for example, in rabbit and in mouse, utilizing the same peptide fragment.

In addition, the materials for use in the assays of the invention are ideally suited for preparation of a kit. Such a kit may comprise a carrier means being compartmentalized to receive in close confinement one or more container means such as vials, test tubes, and the like. Each of said container means comprises one of the separate elements to be used in the method.

For example, one of said container means may comprise an immunoabsorbent-bound peptide fragment. Such fragment may be bound to a separate solid phase immunoabsorbent or directly to the inner walls of a container. A second container may comprise detectably labeled anti-antibody in lyophilized form or in solution.

The carrier may also contain, in addition, a plurality of containers each of which comprises different, predetermined and known amounts of antibody. These latter containers can then be used to prepare a standard curve from which can be interpolated the results obtained from the sample containing the unknown amount of antibody.

In the practice of this invention, the presence of the HTLV-III antibody or the virus itself or portions thereof may be detected in biological fluids and tissues. Any sample containing the unknown amount of HTLV-III antibodies or HTLV-III can be used. Normally, a sample is a liquid such as, for example, urine, saliva, tear drops, cerebrospinal fluid, blood, serum and the like, or a solid or semi-solid such as, for example, tissues, feces, and the like. As is known in the art, the HTLV-III virus and antibodies to the virus are associated with the T-cell disorder, Acquired Immune Deficiency Syndrome (AIDS) and pre-AIDS conditions, such as AIDS-related complex (ARC). In addition, it is also known in the art that antibodies to HTLV-III may be present in a human's or animal's biological fluids or tissue, without such human or animal suffering from AIDS or ARC.

The peptide fragments according to this invention may also be used as a vaccine against the HTLV-III virus. The peptide fragment may be prepared and administered to an animal, as is generally known in the art, to stimulate the production of antibodies. Preferably, the vaccinia virus can be used according to known means for the preparation of HTLV-III vaccines.

The following examples further describe the materials and methods used in carrying out the invention. The examples are not intended to limit the invention in any manner.

### Examples

For the following Examples, the following human sera was used in evaluating the immunoreactivity of the recombinant peptide fragments. Sera were obtained from clinically diagnosed AIDS or AIDS-related complex patients or controls. Each serum was tested under code for HTLV-III/LAV gp 160/120 antibody (L.W. Kitchen et al, Nature, 312: 367-369 (1984)). gp 160/120 antibody was detected in sera from every AIDS patient, but not in any sera from controls. Controls were mostly low risk individuals without clinical signs of AIDS. They included males and females, individuals from Southern, Eastern, and Western U.S. cities and Haiti; rheumatoid factor positive persons; nuclear antibody (ANA) positive people, multiparous women; and a variety of cancer patients. AIDS patients were predominantly homosexuals but also included a few women and children and some transfusion associated cases. The AIDS patients had the same geographical distribution as the controls.

### Example 1

### Plasmid Preparation

### Preparation of Plasmids pJLB0, pJLB1, and pJLB2 from pJLA16

Figure 1 shows the preparation of the plasmids pJLB0, pJLB1, and pJLB2 from the plasmid pJLA16. The construction of the plasmid pJLA16 is described in Lautenberger et al, Gene Anal. Tech., 1:63-66 (1984). The pJLA16 plasmid contains the bacteriophage lambda P_{L} promoter (P_{L}), and both the Shine Dalgarno sequence and leader peptide from the bacteriophage lambda C_{II} gene. The three expression plasmid vectors, pJLB0, pJLB1, and pJLB2 were prepared by digestion of pJLA16 with the restriction endonuclease NruI. After digestion, three BamHI linkers of varying lengths were ligated to the cut plasmid. This process places one BamHI restriction site at the end of the C_{II} bacterial leaders in each of the translation reading frames.

### Preparation of Plasmids with Translation Terminators

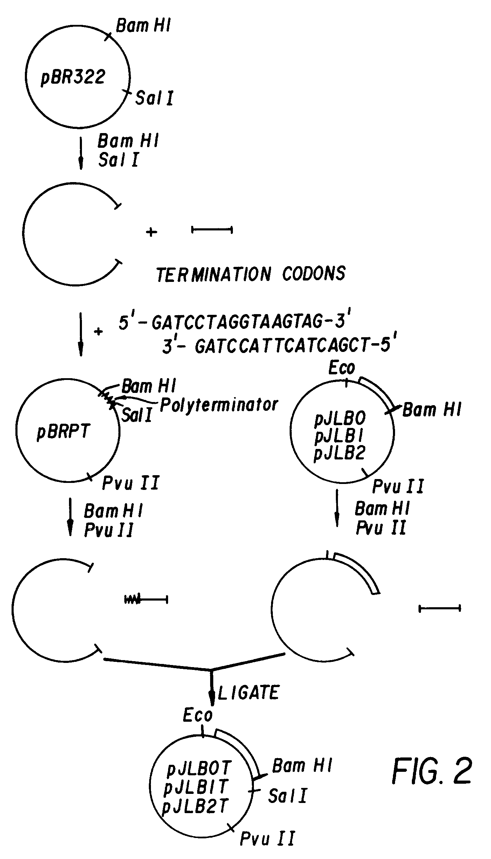

Referring to Figure 2, a synthetic oligonucleotide, containing translation terminators in all three reading frames, was cloned initially into plasmid pBR322 and then, shuttled into the three expresion vectors behind the BamHI cloning site. The resulting plasmids were designated pJLB0T, pJLB1T, and pJLB2T.

### Preparation of Plasmids to Remove a Repressor of Transcription

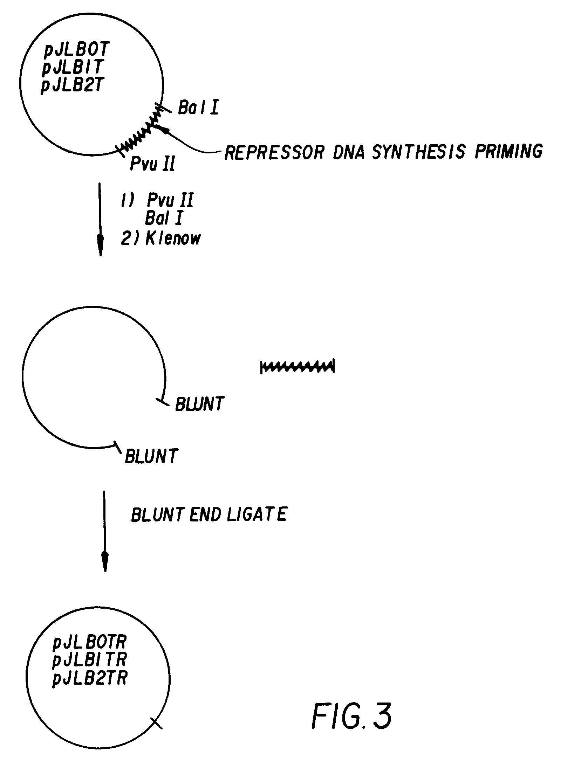

Figure 3 shows removal of the portion of the plasmid vector that encodes a repressor of transcription required for priming of DNA synthesis. The plasmids, pJLBOT, pJLB1T, and pJLB2T, were digested with the restriction endonucleases PvuII and BalI and blunt ended with Klenow fragment. The blunt ends were ligated, and the resulting plasmids were designated pJLB0TR, pJLB1TR, and pJLB2TR.

### Example 2

### Construction of Intermediate Clones for pL-CII Expression System

The following described plasmid series pLCBCO and pLCBC00 were constructed in a manner similar to that for pJLA16. These constructions are described in Shimatake, H. et al., Nature, 292:128 (1981); Oppenheim, A.B. et al., J. Mol. Biol., 158:327 (1982); Lautenberger, J.A. et al., Gene, 23:75 (1983); and Lautenberger, J.A. et al., Gene Anal. Tech., 1:63 (1984).

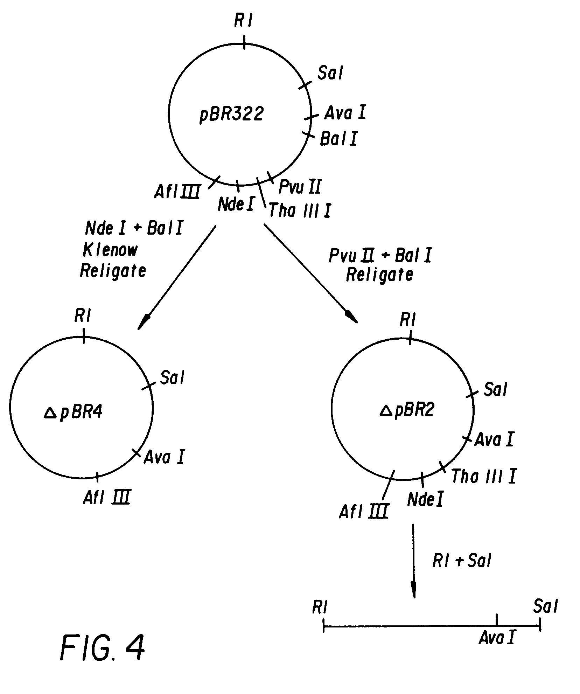

Figure 4 shows the construction of plasmids delta pBR2 and delta pBR4 from pBR322. In plasmid delta pBR2, the repressor of initiation of DNA synthesis is removed by digesting pBR322 with restriction endonucleases PvuII and BalII, then religating the plasmid. Plasmid delta pBR4 was constructed by removing the Nde I site from pBR322 by digestion with restriction endonucleases Nde I and Bal I and the ends blunt ended with Klenow fragment. The blunt ends were ligated and the resulting plasmid designated delta pBR4.

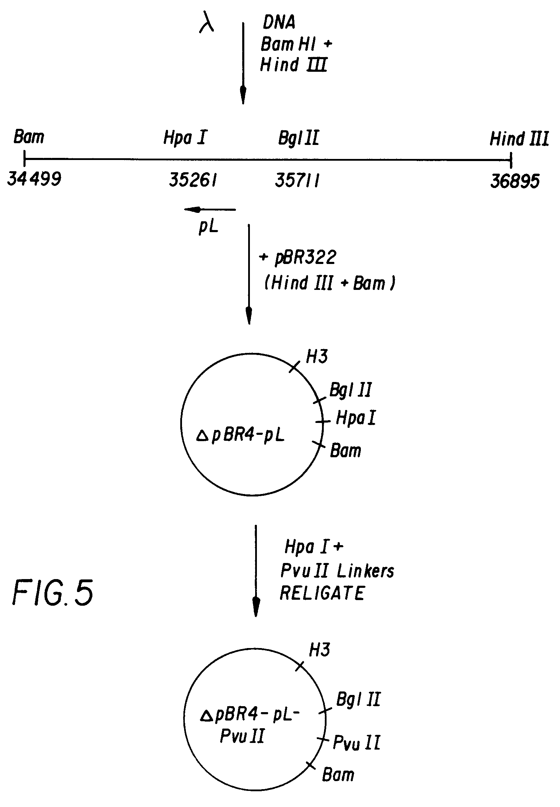

Figure 5 shows the construction of the subcloning of the lambda pL fragment into the delta pBR4 plasmid, and the resulting plasmid delta pBR4-pL. The Hpa I site of delta pBR4-pL was converted to a Pvu II site, by digestion with restriction endonuclease Hpa I and ligating the plasmid with Pvu II linkers.

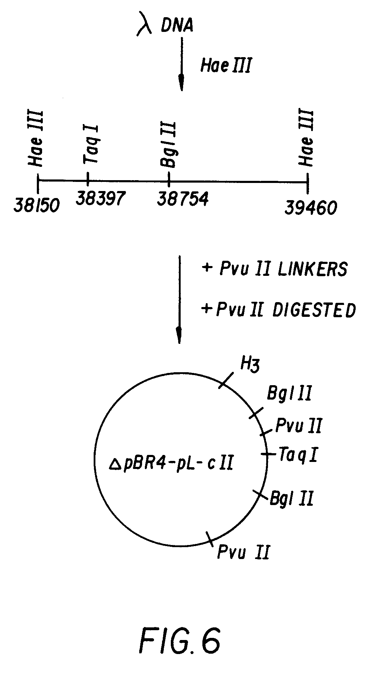

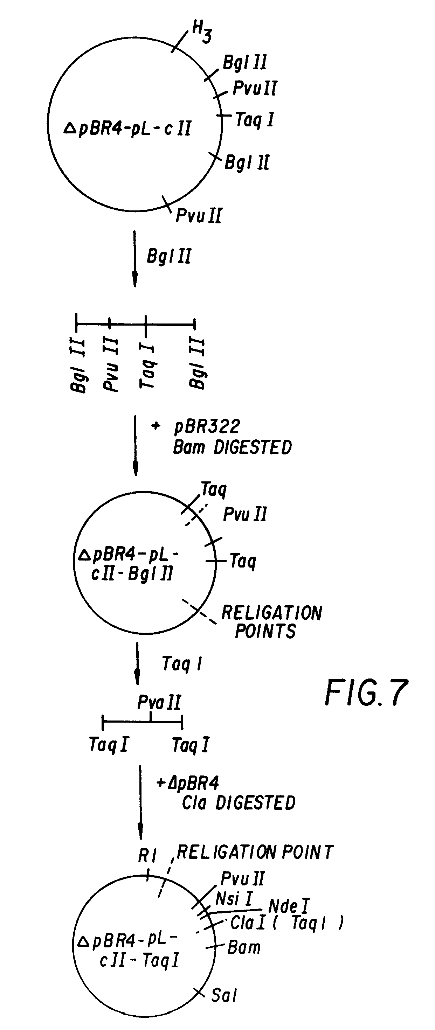

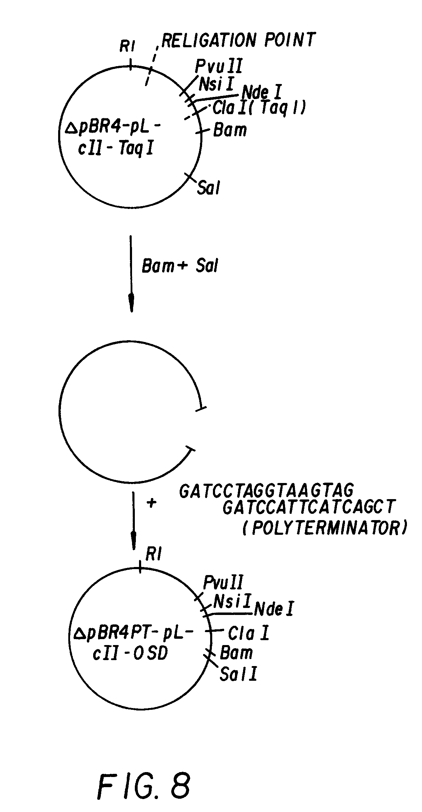

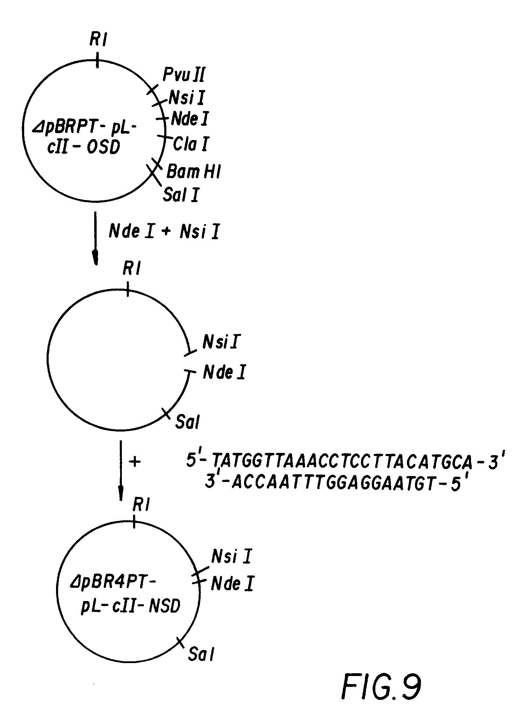

Figure 6 shows the subcloning of the lambda cII Shine-Dalgarno sequencing and coding sequence behind the pL promoter, resulting in plasmid delta pBR4-pL-cII. This plasmid was used in the two step subcloning of essential elements of expresion system as Taq 1 fragments. This construction is shown in Figure 7. The resulting plasmid, delta pBR4-pL-cII-Taq I was then digested with the restriction endonucleases Bam and Sal I. After digestion, the translation polyterminator was added, as shown in Figure 8. The resulting plasmid was designated delta pBR4PT-pL-cII-OSD. "OSD" designates the "old Shine-Dalgarno" sequence. The "OSD" plasmid was modified then, in parallel with the plasmid, delta pBRPT-pL-cII-NSD, which was constructed from the "OSD" plasmid, but contains "new Shine-Dalgarno" sequences, as shown in Figure 9.

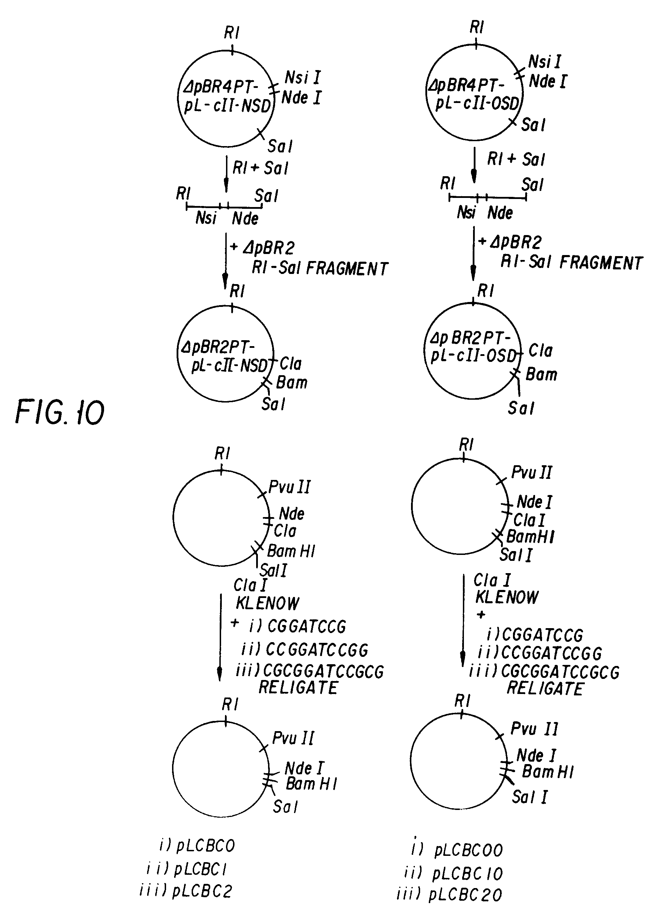

The expression sequences of the "OSD" and the "NSD" plasmids were subcloned, respectively, into plasmid delta pBR2, as shown in Figure 10. The Cla I site on the resulting plasmids were converted to a BamH1 site, to be used for insertion of the gene fragment to be expressed. The BamH1 site is present in a different reading frame for each of the three vectors constructed. Plasmids pLCBCO, pLCBC1, and pLCBC2 contain the new Shine-Dalgarno sequence. Plasmids pLCBC00, pLCBC10, and pLCBC20 are identical to the above-mentioned plasmids, but contain the original cII Shine-Dalgarno sequence.

### Example 3

### Random Cloning and Direct Cloning Strategy

Expressing clones were generated using a random cloning strategy and a direct cloning strategy.

### Random Cloning Strategy

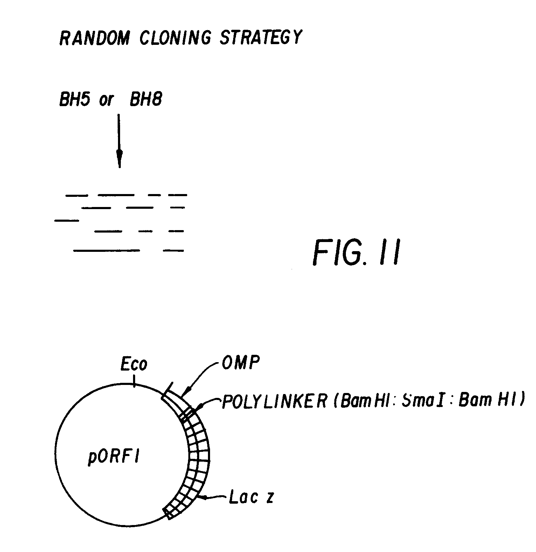

For the random cloning strategy, the DNA of two HTLV-III subclones, BH5 and BH8 was partially digested with either AluI or HaeIII and the ends of the resulting fragments randomized with respect to reading frames by a brief digestion with Bal31 exonuclease. These fragments were then cloned into the Sma1 site of plasmid pORF1 (Weinstock et al, Proc. Nat'l. Acad. Sci., 80:4432-4436 (1983)). (pORF₁ and pORF₂ are deposited in the American Type Culture Collection under ATCC Nos. 39147 and 39145, respectively.) p0RF1 contains DNA sequences encoding an OMP promoter and leader peptide, a polylinker, and beta-galactosidase. The random cloning strategy is depicted in Figure 11. The reading frame for beta-galactosidase is different than that for the OMP-leader peptide. Functional beta-gal (indicated as blue colonies on X-gal plates) will be produced only if a DNA fragment is cloned that contains an open reading frame and realigns the OMP + beta-gal frames. The protein encoded by this cloned DNA must also be expressed as part of an OMP-beta-gal tripartate fusion protein.

The randomized fragments cloned into the Sma1 digested p0RF1 transformed E. coli, MH3000, hosts, were grown on X-gal plates. Blue and white colonies appeared, and the blue colonies selected. Protein extracts were prepared from the blue colonies. Five positive clones were obtained after evaluating the protein extract using Western blot analysis with AIDS patient sera. The BamHI fragments were isolated from each of these five clones and the fragments cloned into pJLB0. The protein produced from this clone was analyzed by Western immunoblot using AIDS patient sera.

### Direct Cloning Strategy

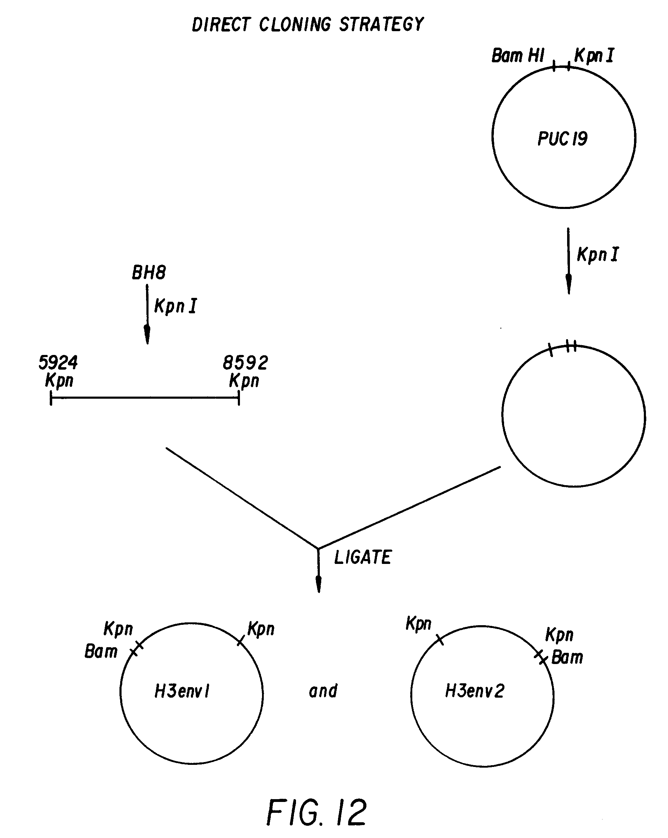

For the direct cloning strategy, two additional subclones were prepared by subcloning the Kpn1 fragment of BH8, nucleotides 5924-8592, into PUC 19. (Yanish-Perron, et al., Gene, 26:101-106 (1983) (PUC 19 is deposited in the American Type Culture Collection under ATCC No. 37254). H3env1, one of the resulting subclones, has the BamH1 site of PUC 19 adjacent to the Kpn1 site, nucleotide no. 5929 in BH8, and H3env2 has the BamH1 site adjacent to the other Kpn1 cite, nucleotide no. 8597. The direct cloning strategy is shown in Figure 12.

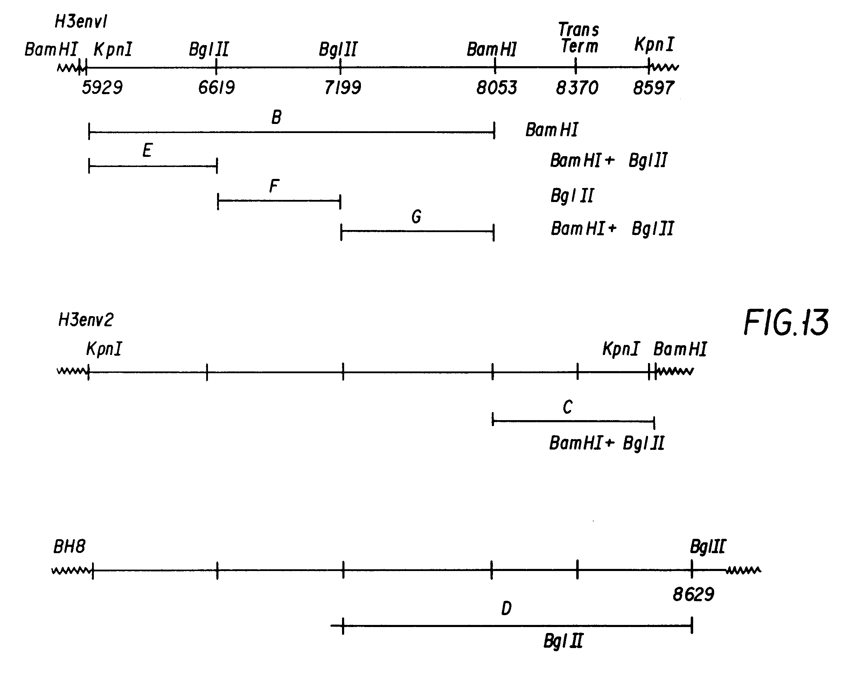

A number of restriction fragments were isolated after digestion of H3env1, H3env2, and BH8 with BamH1 and/or BglII. These restriction fragments are depicted in Figure 13. These fragments were cloned into pJLB0T, pJLB1T, or pJLB2T as appropriate for proper reading frame alignment. Translation terminated with the HTLV termination codon for clones C and D, and in the polyterminator for all other clones. Protein expression and immunoreactivity was assayed by gel electrophoresis and Western blot.

### Example 4

### Immunoreactive HTLV-III Expressing Clones

Table I lists the HTLV-III nucleotide fragments that were cloned for expression of HTLV-III peptides in the appropriate vector from the pJL plasmid series. Peptides produced by expressing clones were screened for immunoreactivity against antibodies to the HTLV-III virus from human patient sera. Immunoreactivity with expressed protein from envelope (env) clones A and E was barely detectable with the strongest sera tested and therefore these clones were not tested further. Clones B, C, and D were severely toxic to the host cells, E. coli. Since expressed HTLV-III protein from these clones was barely detectable with the strongest sera, they were not tested further.

**TABLE I**

| HTLV III Expressing Clones | | | | |
|---|---|---|---|---|
| Name | HTLV-III Nucleotides | Source: Random or Direct | Screened for Immunoreactivity | Toxic to Host Cell |
| Gag 1 68-10 | 653-1218 | Random; BH5 | + | - |
| Pol 1 60-3 | 2600-3911 | Random; BH5 | + | - |
| Pol 2 57-2 | 2743-4211 | Random; BH5 | + | - |
| A;7.1 | 5928-6986 | Random; BH8 | - | - |
| | | | Low immunoreactivity | |
| B;60-25 | 5929-8052 | Direct; H3env1 | - | + |
| C | 8053-8596 | Direct; H3env2 | - | + |
| D;59-9 | 7199-8628 | Direct; BH8 | - | + |
| E;66-1 | 5929-6618 | Direct; H3env1 | - | - |
| | | | Low immunoreactivity | |
| F;60-39 | 6619-7198 | Direct; H3env1 | + | - |
| G;66-21 | 7199-8052 | Direct; H3env1 | + | - |

Table I shows the screening results of expressed protein from various clones for immunoreactivity against antibodies to the HTLV-III virus in human patient sera. The first column identifies the name given to the clone, with the second column designating the nucleotide sequence which encodes the expressed peptide fragment. The third column shows the cloning strategy for constructing the clones and the starting material. In the fourth column, the plus sign (+) indicates that expressed protein was screened for immunoreactivity against a panel of HTLV-III positive sera, while those with a minus sign (-) were not screened because of low immunoreactivity with a high titre AIDS sera or because of severe toxicity to the host bacteria. The last column shows which clones were toxic to the transformed host cell; again, the plus sign (+) indicates toxicity to the host cell, while the minus sign (-) indicates that the clones were not toxic to the host cells.

### Example 5

### Sera Evaluation of HTLV-III Clones

HTLV-III peptides from five expressing clones (gag, Pol 1, Pol 2, env F, and env G) were tested for their ability to detect antibodies to HTLV-III virus in human sera. These clones are described in Table I and the experimental results are listed in Table II. Sera tested included both sera positive for the AIDS HTLV-III virus (+) and sera negative for the AIDS HTLV-III virus (-). All sera were characterized based on radioimmune precipitation. The clones were analyzed by Western blot analysis. None of the sera without AIDS HTLV-III antibody reacted with the clones. However, all sera positive for AIDS HTLV-III antibody reacted with envelope (env) G clone.

**TABLE II**

| Evaluation of HTLV-III Clones | | |
|---|---|---|
| Clone | Sera Tested | No. Sera Reacting with Clone |
| gag | 58 + | 33 +, 25 - |
| | 2 - | 2 - |
| pol 1 | 17 + | 14 +, 3 - |
| | 2 - | 2 - |
| pol 2 | 17 + | 14 +, 3 - |
| | 2 - | 2 - |
| env F | 58 + | 37 +, 21 - |
| | 2 - | 2 - |
| env G | 91 + | 91 + |
| | 87 - | 87 - |

HTLV-III peptides from five expressing clones (gag, Pol 1, Pol 2, env F, and env G) were tested for their ability to detect antibodies to HTLV-III virus in human sera. The expressing clones evaluated are given in the first column, and were previously described in the first and second columns of Table I.

In the second column, the first number, followed by a plus sign (+), is the number of patient's sera tested which were known to be positive for antibodies to HTLV-III. The second number, followed by a minus sign (-), is the number of sera tested which were known to be negative, or which did not contain antibodies to HTLV-III.

The third column shows both the specificity and sensitivity of the peptides' ability to detect antibodies to HTLV-III. The best results are shown with clone env G wherein all 91 positive sera tested gave true-positive results and all 87 negative sera tested gave true-negative results. The next two best results are shown with clones pol 1 and pol 2. With these clones, of the 17 positive sera tested, a true-positive result was given in 14 tests, while a false-negative was given in 3 tests. Both negative sera gave true-negative results. With the first clone, gag, of the 58 positive sera tested, a true-positive result was given in 33 tests, while a false-negative result was given in 25 tests. Both negative sera gave true-negative results. The fourth clone, env F, gave similar results.

### Example 6

### Modification for Increased Expression

In order to increase the expression levels of clone G, two different approaches were taken: i) deleting the DNA encoding two hydrophobic regions of the clone G protein; and ii) fusing the clone G sequences to other HTLV III sequences that are expressed at high levels.

Clone G contains HTLV-III nucleotides 7199 to 8052. The two hydrophobic regions are approximately encoded by 7350 to 7418 (5') and 7851 to 7916 (3'). The 3' hydrophobic region was removed by linearizing H3env1 with BamH1 and removing approximately 200 nucleotides with Bal31 exonuclease. BamH1 linkers were added, plasmids recircularized with DNA ligase. Clones were selected, and designated G-8, G-12, G-29, G-44, G-42, G-46, G-56, G-71, G-79, G-82, and G-84. The new 3' end points were mapped and ranged from 7500 to 7900. The HTLV-III sequences from these clones were subcloned into expression vector pJLB2T and expression levels determined.

The 5' hydrophobic region was removed by M-13 site directed mutagenesis using oligonucleotide 5' TTGTCTGGCCTGTCCTATTCCCAC-3'. This oligo is complementary to nucleotides 7338-7349 and 7419-7430 and will result in the deletion of 23 codons. The deleted DNA sequences are bp 7350-7418, inclusive. A properly constructed clone (delta G) was confirmed by hybridization and DNA sequencing and expression level determined.

Double deletions were also constructed between G-8 and G-71. G-8 and G-71 were chosen since their 3' end points (7840 ± 20 and 7810 ± 20) map near the beginning of the 3' hydrophobic domain. These double deletions were called delta G-8 and delta G-71 and their expression levels determined.

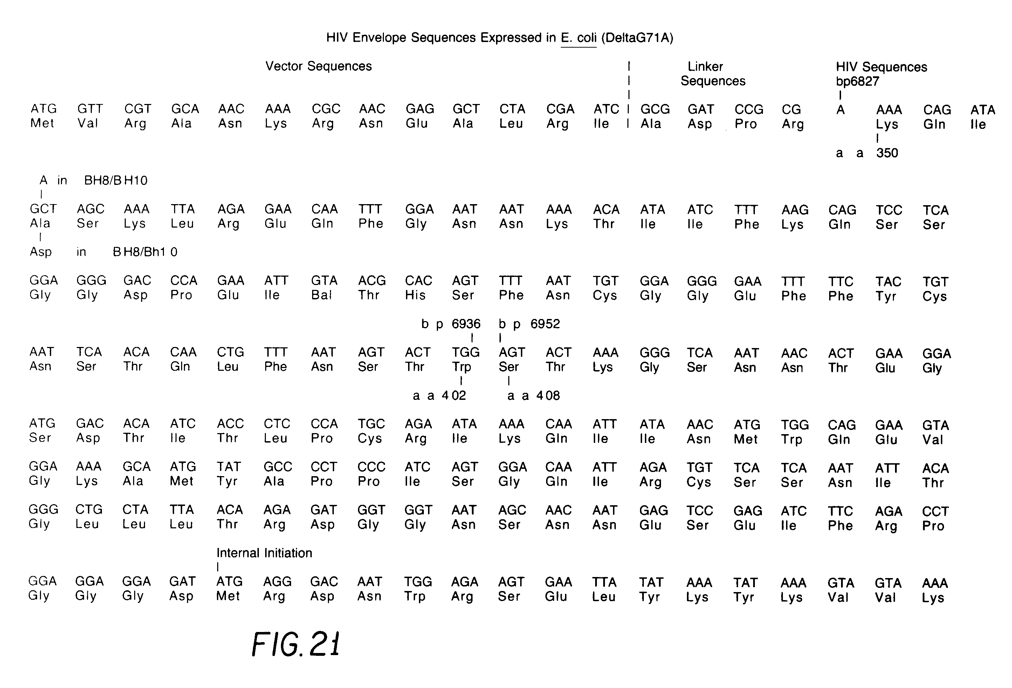

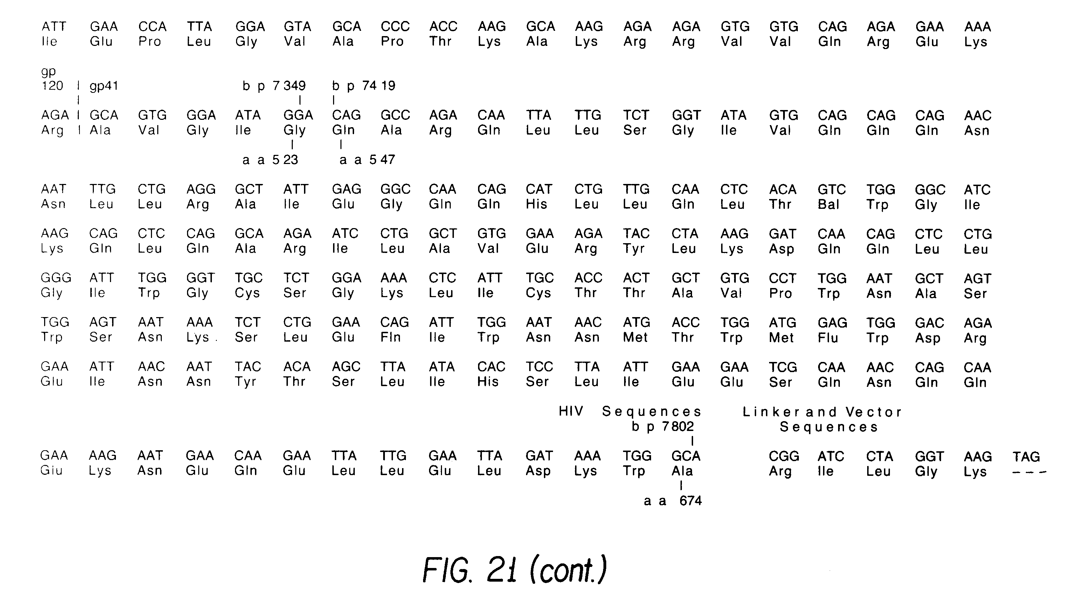

Clone G was extended in the 5' direction to the AhaIII site at nucleotide no. 6827. A BamH1 linker was added at the AhaIII site and expression analyzed (G-A). Double deletions with the Aha extension were also constructed and expression analyzed (delta G-71A and delta G-8A). The amino acid sequence coded by the clone delta G-71A was determined and appears in Figure 21. The actual amino acid sequence coded by the clone delta G-71A is from about bp 6827-6936, 6952-7349, 7419-7802, excluding the regions bp 6937-6951 and 7350-7418. The amino acid peptide sequence is as follows:

AAA CAG ATA
Lys Gln Ile

GCT AGC AAA TTA AGA GAA CAA TTT GGA AAT
Ala Ser Lys Leu Arg Glu Gln Phe Gly Asn

AAT AAA ACA ATA ATC TTT AAG CAG TCC TCA
Asn Lys Thr Ile Ile Phe Lys Gln Ser Ser

GGA GGG GAC CCA GAA ATT GTA ACG CAC AGT
Gly Gly Asp Pro Glu Ile Bal Thr His Ser

TTT AAT TGT GGA GGG GAA TTT TTC TAC TGT
Phe Asn Cys Gly Gly Glu Phe Phe Tyr Cys

AAT TCA ACA CAA CTG TTT AAT AGT ACT TGG
Asn Ser Thr Gln Leu Phe Asn Ser Thr Trp

AGT ACT AAA GGG TCA AAT AAC ACT GAA GGA
Ser Thr Lys Gly Ser Asn Asn Thr Glu Gly

ATG GAC ACA ATC ACC CTC CCA TGC AGA ATA
Ser Asp Thr Ile Thr Leu Pro Cys Arg Ile

AAA CAA ATT ATA AAC ATG TGG CAG GAA GTA
Lys Gln Ile Ile Asn Met Trp Gln Glu Val

GGA AAA GCA ATG TAT GCC CCT CCC ATC AGT
Gly Lys Ala Met Tyr Ala Pro Pro Ile Ser

GGA CAA ATT AGA TGT TCA TCA AAT ATT ACA
Gly Gln Ile Arg Cys Ser Ser Asn Ile Thr

GGG CTG CTA TTA ACA AGA GAT GGT GGT AAT
Gly Leu Leu Leu Thr Arg Asp Gly Gly Asn

AGC AAC AAT GAG TCC GAG ATC TTC AGA CCT
Ser Asn Asn Glu Ser Glu Ile Phe Arg Pro

GGA GGA GGA GAT ATG AGG GAC AAT TGG AGA
Gly Gly Gly Asp Met Arg Asp Asn Trp Arg

AGT GAA TTA TAT AAA TAT AAA GTA GTA AAA
Ser Glu Leu Tyr Lys Tyr Lys Val Val Lys

ATT GAA CCA TTA GGA GTA GCA CCC ACC AAG
Ile Glu Pro Leu Gly Val Ala Pro Thr Lys

GCA AAG AGA AGA GTG GTG CAG AGA GAA AAA
Ala Lys Arg Arg Val Val Gln Arg Glu Lys

AGA|GCA GTG GGA ATA GGA CAG GCC AGA CAA
Arg|Ala Val Gly Ile Gly Gln Ala Arg Gln

TTA TTG TCT GGT ATA GTG CAG CAG CAG AAC
Leu Leu Ser Gly Ile Val Gln Gln Gln Asn

AAT TTG CTG AGG GCT ATT GAG GGC CAA CAG
Asn Leu Leu Arg Ala Ile Glu Gly Gln Gln

CAT CTG TTG CAA CTC ACA GTC TGG GGC ATC
His Leu Leu Gln Leu Thr Bal Trp Gly Ile

AAG CAG CTC CAG GCA AGA ATC CTG GCT GTG
Lys Gln Leu Gln Ala Arg Ile Leu Ala Val

GAA AGA TAC CTA AAG GAT CAA CAG CTC CTG
Glu Arg Tyr Leu Lys Asp Gln Gln Leu Leu

GGG ATT TGG GGT TGC TCT GGA AAA CTC ATT
Gly Ile Trp Gly Cys Ser Gly Lys Leu Ile

TGC ACC ACT GCT GTG CCT TGG AAT GCT AGT
Cys Thr Thr Ala Val Pro Trp Asn Ala Ser

TGG AGT AAT AAA TCT CTG GAA CAG ATT TGG
Trp Ser Asn Lys Ser Leu Glu Fln Ile Trp

AAT AAC ATG ACC TGG ATG GAG TGG GAC AGA
Asn Asn Met Thr Trp Met Flu Trp Asp Arg

GAA ATT AAC AAT TAC ACA AGC TTA ATA CAC
Glu Ile Asn Asn Tyr Thr Ser Leu Ile His

TCC TTA ATT GAA GAA TCG CAA AAC CAG CAA
Ser Leu Ile Glu Glu Ser Gln Asn Gln Gln

GAA AAG AAT GAA CAA GAA TTA
Glu Lys Asn Glu Gln Glu Leu

TTG GAA TTA GAT AAA TGG GCA
Leu Glu Leu Asp Lys Trp Ala

Delta G-71A and delta G-8A were also fused to the pol expressing clone, 57-2 as both env-pol and pol-env fusions. HTLV-III DNA fragments from all constructs were subcloned into a plasmid from the pJL expression vector series. E. coli hosts, MZl, were used to express the peptide fragments. HTLV-III sequences from delta G-71A were then cloned and expressed in three expression vectors: pJLBOT, pLCBCO, and pLCBCOO. These clones were designated p-delta-G71A8, p-delta-G71ACN, and p-delta-G71AC. All three clones produced the same protein at the same levels of expression shown in Table III for delta G-71A. p-delta-G71AC is deposited with the ATCC in bacterial host MZl.

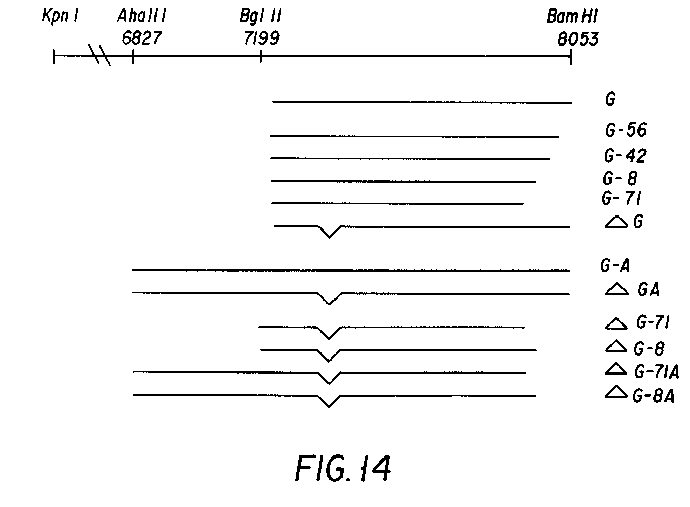

All constructs prepared and analyzed are shown in Figure 14. Relative expression levels are shown in Table III. The highest expression levels were seen in clones delta G-8, delta G-71, delta G-8A, and delta G-71A.

In summary, neither single deletion or Aha extension increased expression levels significantly. However, both double deletions (delta G-71 and delta G-8) and the Aha extension of these double deletions did dramatically increase expression levels. Pol-delta G-71 fusions produced barely detectable protein levels.

**TABLE III**

| SUMMARY OF EXPRESSION LEVELS^{*} | |
|---|---|
| Clone | Estimated Expression Level (% of total bacterial protein) |
| G, G-8, G-12, G-29, G-44 | <0.02% |
| G-42, G-46, G-56, G-71, G-79 | |
| G-82, G-84, delta G, GA, delta G-A | |
| delta G-8, delta G-71 | 5-10% |
| delta G-8A, delta G-71A | |
| delta G-71A:pol | <0.005% |
| pol:delta G-71A | |

| | |
|---|---|
| ^{*} See Figure 14 for maps of the noted clones. | |

### Example 7

### Immunoreactivity of Clones

Immunoreactivity of all clones was tested as follows:
Bacterial cultures were grown in L Broth at 32°C until A₅₅₀=0.4. Cultures were then grown at 42°C for 1 hour. A₅₅₀ readings were taken and the bacteria harvested by centrifugation. SDS gel loading buffer was added (0.1 ml per 1.5 ml of A₅₅₀=0.5) and samples placed in a boiling water bath for 5 minutes. Proteins were separated by SDS polyacrylamide gel electrophoresis, blotted onto nitrocellulose and analyzed by reacting with a high titre sera from an AIDS patient, or for screening purposes with sera independently analyzed for HTLV-III antibodies by radioimmune precipitation.

### Example 8

### Protein Purification

High-level expression of desired proteins in bacteria typically results in the formation of insoluble inclusion bodies or aggregates. In order to purify the expressed, recombinant proteins, the solubility of these proteins must be increased. Recombinant protein delta G-71A from HTLV-III expressing clones p-delta-G71A8 or p-delta-G71AC was solubilized and purified as follows:
A culture of E. coli delta G71A was grown in LB Broth at 32°C to an A₅₅₀ = 0.5. The expression of HTLV-III antigens was induced by temperature shift of the culture to 42°C, and incubated at 42° for 90 minutes. The cells were collected by centrifugation at 4000 x g for 30 minutes, washed once with TBS and resuspended in 50 mM Tris HCl pH 8.5, containing 1 mM PMSF, 50 mM DTT, 0.2% Triton® X-100, and 10 mM EDTA. The cells were then lysed by enzymatic digestion with lysozyme and brief sonications. The expressed antigen existed as insoluble aggregates and could be collected by centrifugation at 5000 x g for 30 minutes. These aggregates were washed several times with mild detergents, and 6 M urea. The protein aggregates were solubilized with 8 M urea in 50 mM TrisHCl, pH 8.5, containing 1% beta-mercaptoethanol. The free sulfhydryls were alkylated with a 10-fold excess of iodoacetic acid at room temperature. Partial purification of the alkylated antigen was achieved by gel filtration on a Sepharose® 6B-CL column operated in the presense of 8 M urea.

The partially purified antigens were then subjected to citraconylation in borate buffer, pH 8.5, containing 8 M urea. This was done by treating the antigen preparation with a 50-fold molar excess of citraconic anhydride at room temperature for 90 minutes. The citraconylated sample was dialyzed extensively against 0.1 M borate buffer, pH 8.5, and the antigen was further purified by gel filtration on a Fractogel® column, equilibrated and eluted with the same buffer.

The citraconylated antigen was eluted from the column in a position corresponding to the elution position of a protein standard with a molecular weight of 35,000, suggesting that the antigen existed in this buffer system as a soluble, monomeric molecule. The fractions devoid of the other protein contaminations were pooled and used for ELISA.

The citraconylated antigen could be adsorbed onto microtiter plates and the immobilized antigen remained immunoreactive toward HTLV-III antibodies following deblocking of amino groups.

The citraconylated amino groups of the immobilized antigen could be hydrolyzed by the addition of 0.1 N acetic acid into each well. Enzyme-linked immunoassay (ELISA) revealed that the immunoreactivity of the deblocked molecules was considerably higher than that of the blocked (citraconylated) molecules as judged by the higher absorbancy value obtained for the deblocked protein under identical assay conditions.

### Example 9

### ELISA Development

All materials used for ELISA development other than the recombinant protein were provided by Ortho Diagnostics Systems, Inc. Purified delta G-71A was adjusted to a final protein concentration of 3.0 ug/ml in 2M guanidine.HCl in 0.05 M Tris.HCl, pH 8.5. An aliquot (0.1ml) of this antigen solution was allowed to bind to Immulon II microtitre wells for 16 hours at 4°C. The remaining protein solution was removed, wells washed with 2M guanidine-HCl, and non-specific binding sites blocked by contact with 0.1 ml of 10% BSA for 2 hours at 37°C. The sera was removed and the wells washed 5 times with PBS containing 0.05% Tween® 20. A mouse monoclonal anti-human IgG conjugated with horseradish peroxidase (HRP) was added and allowed to bind for 1 hour at 37°C. The secondary antibody was removed, wells were washed, and substrate solution containing o-phenylenediamine.HCl (OPD) was added and allowed to react with any bound HRP for 15-30 minutes. The reaction was stopped by the addition of 2M H₂S0₄, and A₄₉₀ read on a Dynatech microtiter plate reader.

Table IV summarizes the ELISA results obtained using the above method. It shows that the ELISA adsorbance values for AIDS and controls did not overlap and that the test reproducibility was acceptable. If a cut-off value of 0.3 was chosen, there would have been no false positives or false negatives in this group of samples.

Using purified recombinant protein delta G-71A and the procedure above, the anti-HTLV-III samples were also tested provided under the code from the College of American Pathologists (Set WM-B). There were three samples from HTLV-III infected persons that had been diluted in serum from non-infected people. These three samples had OD values no less than 0.70, 0.60, and 0.94 on three separate occasions. Two negative samples scored 0.04 and 0.06. Thus the recombinant protein ELISA was able to easily identify the positive samples.

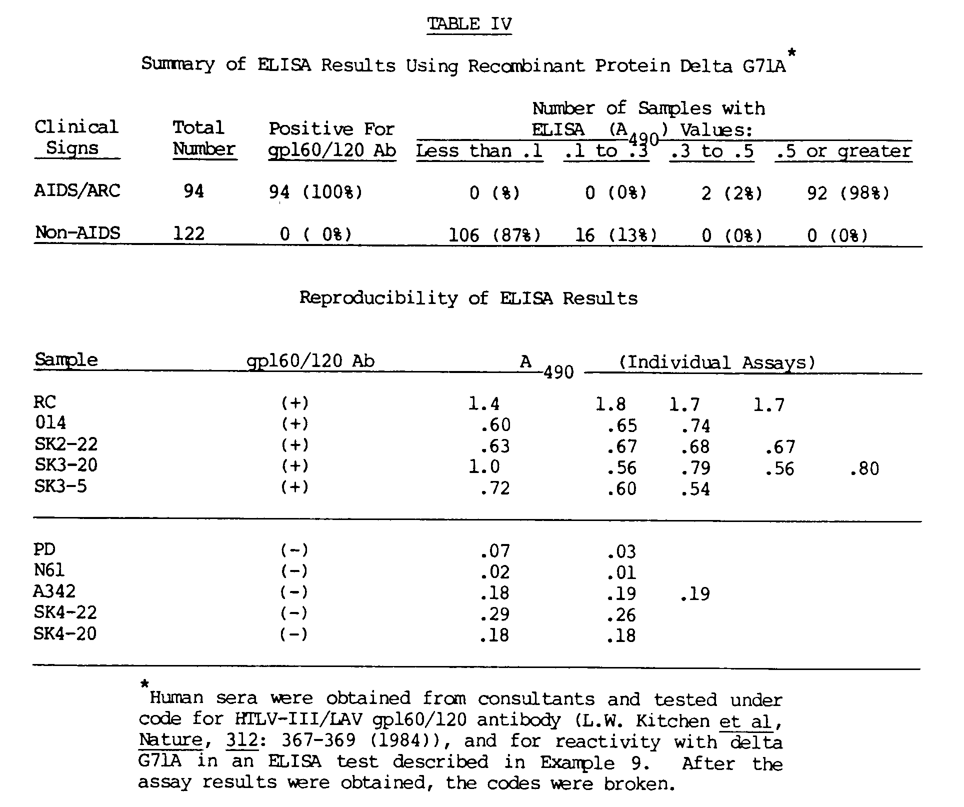

In testing additional sera using the method described, some control samples gave very high A₄₉₀ readings (e.g. 0.60). These samples had no indication of HTLV-III LAV antibody by radioimmune precipitation or Western blots with virus, and were from low risk individuals. Therefore, an improved method was developed.

Purified delta G-71A in 6 M guanidine.HCl, 50 mM Tris, pH 8.0 was diluted to 3ug/ml with 2 M guanidine.HCl, 50 mM Tris, pH 8.0, and 100ul was added to polystyrene wells (e.g. Immulon 2 wells (Dynatech)). The antigen was allowed to adsorb at 4-37°C for 30 minutes to 8 weeks (usually 18 hrs.). The antigen solution was removed from the wells and 100ul per well of 0.15 M NaCl in 10 mM phosphate buffer, pH 7.3 (PBS) plus 4-10% normal goat serum (NRG) (5% preferred) was added. The wells were then incubated at 30-39°C for 30 minutes to 5hr, 2hr being preferred.

The PBS and NRG solution was removed and 100ul per well of 0.5-1.5 M NaCl, 10 mM EDTA 10 mM Tris, pH 8.0 (TBS) was added. 5ul of sera were added and incubated at 18-39°C (preferably 37°) for 15 minutes to 3 hours (1 hr being preferred). The wells were aspirated to waste, and washed 4 to 5 times with 0.05% Tween® 20 in deionized water. To each well 100ul of mouse monoclonal anti-human IgG conjugated to horseradish peroxidase (Ortho) were added and incubated for 15 min to 1 hour at 20°C to 39°C. The wells were emptied and washed 4 to 5 times with 0.05% Tween® 20 in deionized water. o-Phenylenediamine.2HCl substrate in citrate phosphate buffer (Ortho) (100ul) was added and reacted for 30 minutes at 20-22°C. Sulfuric acid (25ul of a 2M solution) was added, and the A₄₉₀ read on a Dynatech microplate reader.

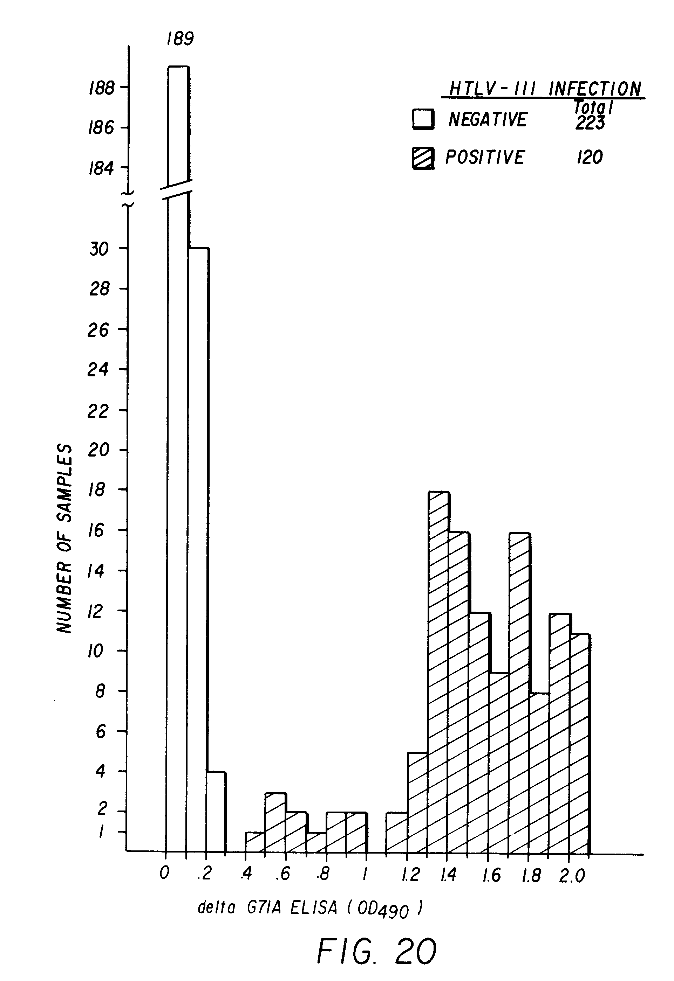

The results with 223 control and 120 AIDS samples are shown in Figure 20. The highest control value was 0.25 and the lowest AIDS sample value was 0.49. Thus, there was no overlap between control and AIDS specimens. All the sera used in Example 9 are included in this set. The mean and standard deviation of the control group was 0.05 ± 0.05. Included in the control group were samples which were false positives on a commercial virus antigen ELISA. More importantly, there were at least 10 AIDS samples, confirmed to be HTLV-III/LAV antibody positive by radioimmune precipitation and Western blots with virus, which were negative by virus ELISA (5 of the samples had an OD less than 0.05, all 10 samples were less than .15) but positive by recombinant ELISA (all 10 samples had absorbancies greater than 0.49).

### EXAMPLE 10

### Coating of Microtiter Plates with Citraconylated Recombinant Protein and Use in an ELISA After Deblocking

S-alkylated or free sulfhydryl citraconylated recombinant HTLV-III antigen (50 ul of a 10 ug protein/ml solution in 25-50 mM sodium borate buffer, pH 9.0) were added to each well of a polystyrene microtiter plate, followed by an equal volume of 0.15 M sodium citrate buffer, pH 5.5-5.6. The plates were incubated for a period sufficiently long to completely hydrolyze citraconyl groups from amino groups (16 hours at 37°C or 24 hours at 25°C, as determined by a standard TNBS test for free amino groups. After aspirating the reaction mixture to waste, the non-specific binding sites, on the adsorbed recombinant protein and on the polystyrene surfaces themselves were blocked by incubating each well with a solution of 1% (w/v) pure bovine serum albumin in PBS, pH 7.6, containing 0.06% Tween® 20 detergent. The solution was aspirated to waste, and the wells air dried.

Aliquots of patient's blood sera, appropriately diluted with PBS puffer, pH 7.6, were added to wells, and incubated at 37°C for 1 hour. The excess fluid was aspirated to waste, and the cells washed once with PBS buffer, pH 7.6. Thereafter, horseradish peroxidase-conjugated anti-human IgG was added. The plates were incubated, washed, and the peroxidase, chromagen, and o-phenylenediamine.HCL, added. The absorbancies at 550 nm were determined in a microtiter plate spectrophotometer (Dynatech MR600). The results (Table V) showed that all HTLV-III positive samples (confirmed by immunoblot and radioimmunoprecipitation assays according to L.W. Kitchen et al., Nature 312:166 (1984)) had ELISA absorbancies greater than 0.41, with all but 6 samples greater than 1.00. All HTLV-III-negative samples i.e., background absorbancies, had readings below 0.24. Therefore, setting a cut-off ELISA reading at 0.30, the ELISA test with decitraconylated recombinant HTLV-III/LAV antigen was 100% accurate.

Deblocking of citraconylated recombinant HTLV-III protein antigen was necessary for its full expression of immunoreactivity, although it is clear that even citraconylated protein retains some activity in the ELISA test.

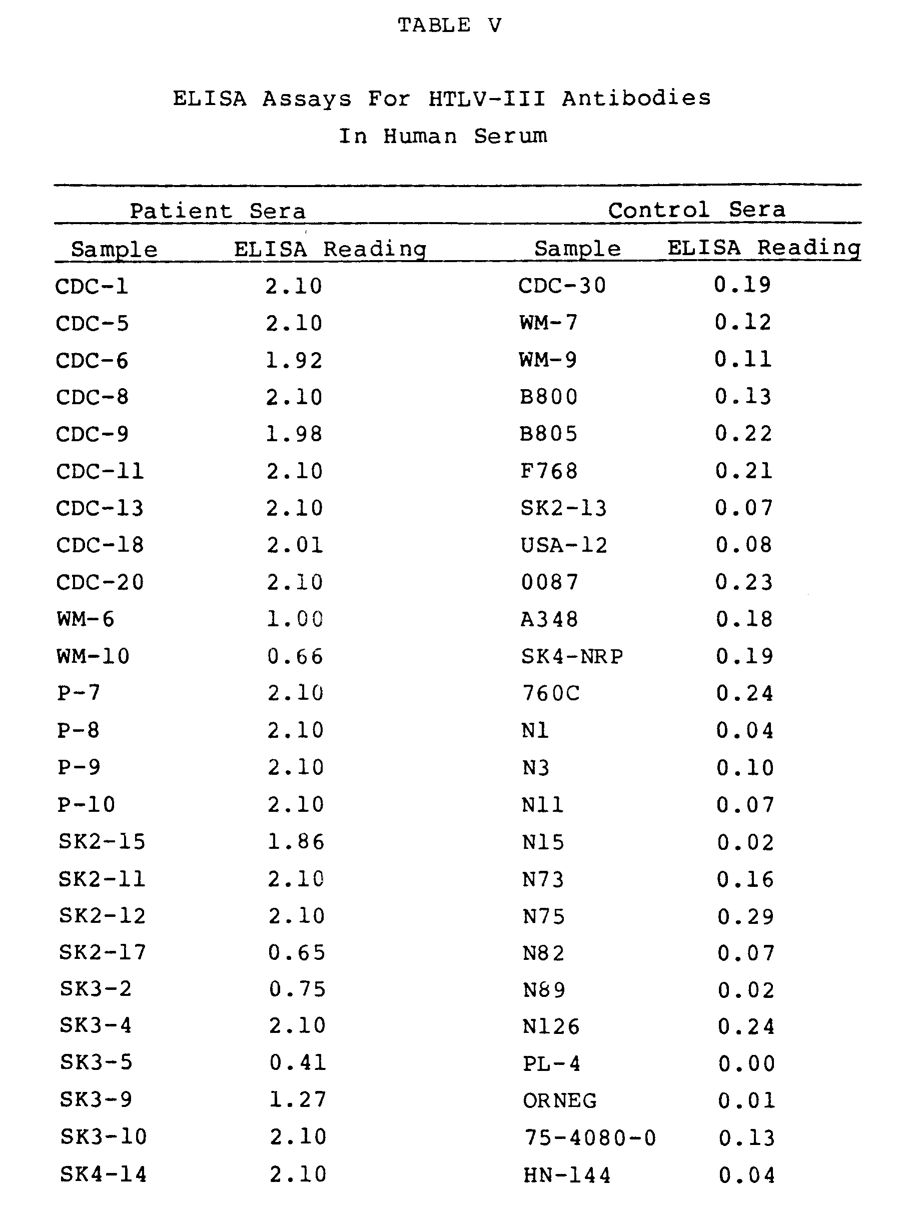

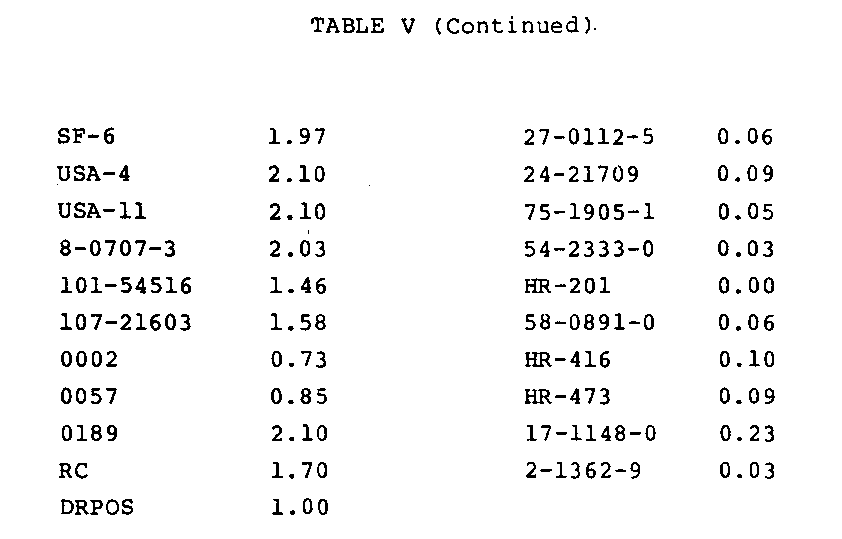

### EXAMPLE 11

### Latex Agglutination Test for Antibodies to the HTLV-III AIDS Virus Protein

Citraconylated recombinant HTLV-III peptide antigen was physically adsorbed to latex beads (2.5% suspension of 0.6 micron diameter beads) in sodium bicarbonate buffer, pH 9.0 during a 16 hour incubation with constant gentle stiring at 25°C. Citraconyl groups were then removed by treatment of adsorbed protein with 0.1M sodium acetate buffer, pH 4.0, for 16 hours at 25°C.

Antigen-coated latex beads were collected by brief centrification at low speed, then resuspended in 1% bovine serum albumin-0.06% Tween® 20 in PBS buffer, pH 7.6, in order to block non-specific binding sites. The final concentration of resuspended beads was 0.6%. Coated beads were stored at 4°C.

The test for antibodies was performed by mixing 25 ul of a patient's serum with 15 ul of coated latex beads on a glass slide, then rotating the slide at 100 RPM for 8 minutes at 25°C.

A positive result for antibodies to the antigen was represented by agglutination of the coated latex beads, said agglutination being visible to the unaided eye. Alternatively, low magnification microscopy was used.

The results of 50 such tests are shown in Table VI. Twenty-two of 24 sera that had been positive by the ELISA test were also positive by the latex bead agglutination (LBA) test. Retests of the two discordant samples were positive when the sera were used in undiluted form. All 24 sera that were negative by ELISA test were also negative by the LBA test. These results indicate that, at a 1:10 dilution of sera, the LBA test is 92% sensitive and 100% specific, whereas using undiluted sera the sensitivity and specificity are both 100%, relative to the ELISA test.

Although the instant disclosure sets forth all essential information in connection with the invention, the numerous publications cited herein may be of assistance in understanding the background of the invention and the state of the art. Moreover, the foregoing invention has been described in some detail by way of illustration and example for purposes of clarity and understanding. It will be obvious that certain changes and modifications may be practiced within the scope of the invention, as limited only by the scope of the appended claims.

**TABLE VI**

| Latex Bead Agglutination Test For HTLV-III/LAV Antibodies In Human Serum* | | | |
|---|---|---|---|
| Patient Sera | | Control Sera | |
| Sample | Result* | Sample | Result* |
| A208004 | 2+ | 26 | NR |
| A20806 | 4+ | 27 | NR |
| A202411 | 4+ | 28 | NR |
| A206210 | 3+ | 29 | NR |
| A206110 | 3+ | 30 | NR |
| A102008 | 3+ | 31 | NR |
| A16103 | 3+ | 32 | NR |
| 003 | 4+ | 33 | NR |
| 014 | 2+ | 34 | NR |
| 017 | 1+ | 35 | NR |
| POS. CONTROL | 3+ | 36 | NR |
| RC | 4+ | 37 | NR |
| P5 | 2+ | 38 | NR |
| P7 | 3+ | 39 | NR |
| P8 | 3+ | 40 | NR |
| P9 | 4+ | 41 | NR |
| P10 | 4+ | 42 | NR |
| 6273 | 2+ | 43 | NR |
| 6316 | 3+ | 44 | NR |
| 6272 | 3+ | 45 | NR |
| 6310 | 3+ | 46 | NR |
| SK2-14 | 3+ | 48 | NR |
| SK2-17 | WR | 48 | NR |
| SK2-21 | WR | 49 | NR |
| | | 50 | NR |
| NR: No reaction WR: Weak reaction undiluted | | | |

| | | | |
|---|---|---|---|
| *Sera diluted 1:10 | | | |
| +Subjective estimation by unaided eyes. | | | |

## Claims (Claims for the following Contracting State(s): BE, FR, DE, IT, GR, NL, SE, CH, LI, LU, GB)

1. A peptide fragment which is immunoreactive to antibodies against the HTLV-III virus and encoded by the HTLV-III provirus nucleotide sequence 7199 to 8052.

2. A peptide fragment which is immunoreactive to antibodies against the HTLV-III virus and encoded by a nucleotide sequence of the HTLV-III provirus at least from about 7199 to about 7810, which excludes hydrophobic regions 7350 to 7418 and 7851 to 7916 and which includes a segment upstream from the amino end, to about nucleotide 6827.

3. A peptide fragment which is immunoreactive to antibodies against the HTLV-III virus and is encoded by a nucleotide sequence of the HTLV-III provirus from about 6827-6936, 6952-7349, 7419-7802, excluding 6937-6951 and 7350-7418.

4. A peptide sequence comprising the amino acid residues shown in the lower of the two lines in the following diagram:

AAA CAG ATA
Lys Gln Ile

GCT AGC AAA TTA AGA GAA CAA TTT GGA AAT
Ala Ser Lys Leu Arg Glu Gln Phe Gly Asn

AAT AAA ACA ATA ATC TTT AAG CAG TCC TCA
Asn Lys Thr Ile Ile Phe Lys Gln Ser Ser

GGA GGG GAC CCA GAA ATT GTA ACG CAC AGT
Gly Gly Asp Pro Glu Ile Bal Thr His Ser

TTT AAT TGT GGA GGG GAA TTT TTC TAC TGT
Phe Asn Cys Gly Gly Glu Phe Phe Tyr Cys

AAT TCA ACA CAA CTG TTT AAT AGT ACT TGG
Asn Ser Thr Gln Leu Phe Asn Ser Thr Trp

AGT ACT AAA GGG TCA AAT AAC ACT GAA GGA
Ser Thr Lys Gly Ser Asn Asn Thr Glu Gly

ATG GAC ACA ATC ACC CTC CCA TGC AGA ATA
Ser Asp Thr Ile Thr Leu Pro Cys Arg Ile

AAA CAA ATT ATA AAC ATG TGG CAG GAA GTA
Lys Gln Ile Ile Asn Met Trp Gln Glu Val

GGA AAA GCA ATG TAT GCC CCT CCC ATC AGT
Gly Lys Ala Met Tyr Ala Pro Pro Ile Ser

GGA CAA ATT AGA TGT TCA TCA AAT ATT ACA
Gly Gln Ile Arg Cys Ser Ser Asn Ile Thr

GGG CTG CTA TTA ACA AGA GAT GGT GGT AAT
Gly Leu Leu Leu Thr Arg Asp Gly Gly Asn

AGC AAC AAT GAG TCC GAG ATC TTC AGA CCT
Ser Asn Asn Glu Ser Glu Ile Phe Arg Pro

GGA GGA GGA GAT ATG AGG GAC AAT TGG AGA
Gly Gly Gly Asp Met Arg Asp Asn Trp Arg

AGT GAA TTA TAT AAA TAT AAA GTA GTA AAA
Ser Glu Leu Tyr Lys Tyr Lys Val Val Lys

ATT GAA CCA TTA GGA GTA GCA CCC ACC AAG
Ile Glu Pro Leu Gly Val Ala Pro Thr Lys

GCA AAG AGA AGA GTG GTG CAG AGA GAA AAA
Ala Lys Arg Arg Val Val Gln Arg Glu Lys

AGA|GCA GTG GGA ATA GGA CAG GCC AGA CAA
Arg|Ala Val Gly Ile Gly Gln Ala Arg Gln

TTA TTG TCT GGT ATA GTG CAG CAG CAG AAC
Leu Leu Ser Gly Ile Val Gln Gln Gln Asn

AAT TTG CTG AGG GCT ATT GAG GGC CAA CAG
Asn Leu Leu Arg Ala Ile Glu Gly Gln Gln

CAT CTG TTG CAA CTC ACA GTC TGG GGC ATC
His Leu Leu Gln Leu Thr Bal Trp Gly Ile

AAG CAG CTC CAG GCA AGA ATC CTG GCT GTG
Lys Gln Leu Gln Ala Arg Ile Leu Ala Val

GAA AGA TAC CTA AAG GAT CAA CAG CTC CTG
Glu Arg Tyr Leu Lys Asp Gln Gln Leu Leu

GGG ATT TGG GGT TGC TCT GGA AAA CTC ATT
Gly Ile Trp Gly Cys Ser Gly Lys Leu Ile

TGC ACC ACT GCT GTG CCT TGG AAT GCT AGT
Cys Thr Thr Ala Val Pro Trp Asn Ala Ser

TGG AGT AAT AAA TCT CTG GAA CAG ATT TGG
Trp Ser Asn Lys Ser Leu Glu Fln Ile Trp

AAT AAC ATG ACC TGG ATG GAG TGG GAC AGA
Asn Asn Met Thr Trp Met Flu Trp Asp Arg

GAA ATT AAC AAT TAC ACA AGC TTA ATA CAC
Glu Ile Asn Asn Tyr Thr Ser Leu Ile His

TCC TTA ATT GAA GAA TCG CAA AAC CAG CAA
Ser Leu Ile Glu Glu Ser Gln Asn Gln Gln

GAA AAG AAT GAA CAA GAA TTA
Glu Lys Asn Glu Gln Glu Leu

TTG GAA TTA GAT AAA TGG GCA
Leu Glu Leu Asp Lys Trp Ala

5. An antibody derived by using a peptide fragment as claimed in any one of claims 1 to 4 as an immunogen.

6. A method for the detection of antibodies against the HTLV-III virus comprising contacting a peptide fragment as in any of claims 1 to 4 with a sample suspected of containing antibodies against the HTLV-III virus and detecting the presence of said antibodies.

7. A method for the detection of the HTLV-III virus or portions thereof comprising contacting a sample suspected of containing the HTLV-III virus or portions thereof with an antibody of claim 5 and detecting the presence of said virus.

8. An expression vehicle comprising a polynucleotide sequence coding for a peptide fragment as claimed in any one of claims 1 to 4.

9. An expression vehicle as claimed in claim 8 which is a plasmid.

10. A plasmid as claimed in claim 9 which is: pJLB0T, pJLB1T, and pJLB2T (as described in Example 1 and depicted in Figures 1 and 2); pJLB0TR, pJLB1TR, and pJLB2TR (as described in Example 1 and depicted in Figure 3); and pLCBC0, pLCBC1, pLCBC2, pLCBC00, pLCBC10, pLCBC20 (as described in Example 2 and depicted in Figures 4 to 10).

11. A host cell transformed with an expression vehicle as claimed in any of claims 8 to 10.

12. A method of expressing a peptide fragment as in any of claims 1 to 4 comprising transforming a host cell with an expression vehicle comprising a polynucleotide sequence coding for said peptide fragment, culturing said transformed host, and expressing said peptide fragment.

13. A vaccine against the HTLV-III virus comprising an effective amount of a peptide fragment as claimed in any one of claims 1 to 4 and an acceptable carrier.

14. The use of a peptide fragment as claimed in any one of claims 1 to 4 in the preparation of a vaccine against the HTLV-III virus.

15. A kit for detecting HTLV-III antibodies comprising a carrier means compartmentalized to receive one or more container means, at least one of said container means containing an immobilized peptide fragment as claimed in any of claims 1 to 4.

16. A kit for detecting the HTLV-III virus or portions thereof comprising a carrier means compartmentalized to receive one or more container means, at least one of said one or more container means containing an antibody of claim 5.

## Claims (Claims for the following Contracting State(s): AT, ES)

1. A process for the preparation of a peptide fragment which is immunoreactive to antibodies against the HTLV-III virus and encoded for by the HTLV-III provirus nucleotide sequence 7199 to 8052, the process comprising coupling amino acids by peptide bond formation.

2. A process for the preparation of a peptide fragment which is immunoreactive to antibodies against the HTLV-III virus and encoded for by a nucleotide sequence of the HTLV-III provirus at least from about 7199 to about 7810, which excludes hydrophobic regions 7350 to 7418 and 7851 to 7916 and which includes a segment upstream from the amino end, to about nucleotide 6827, the process comprising coupling amino acids by peptide bond formation.

3. A process for the preparation of a peptide fragment which is immunoreactive to antibodies against the HTLV-III virus and is encoded for by a nucleotide sequence of the HTLV-III provirus from about 6827-6936, 6952-7349, 7419-7802, excluding 6937-6951 and 7350-7418, the process comprising coupling amino acids by peptide bond formation.

4. A process for the preparation of a peptide sequence comprising the amino acid residues shown in the lower of the two lines in the following diagram: 
AAA CAG ATA
Lys Gln Ile

GCT AGC AAA TTA AGA GAA CAA TTT GGA AAT
Ala Ser Lys Leu Arg Glu Gln Phe Gly Asn

AAT AAA ACA ATA ATC TTT AAG CAG TCC TCA
Asn Lys Thr Ile Ile Phe Lys Gln Ser Ser

GGA GGG GAC CCA GAA ATT GTA ACG CAC AGT
Gly Gly Asp Pro Glu Ile Bal Thr His Ser

TTT AAT TGT GGA GGG GAA TTT TTC TAC TGT
Phe Asn Cys Gly Gly Glu Phe Phe Tyr Cys

AAT TCA ACA CAA CTG TTT AAT AGT ACT TGG
Asn Ser Thr Gln Leu Phe Asn Ser Thr Trp

AGT ACT AAA GGG TCA AAT AAC ACT GAA GGA
Ser Thr Lys Gly Ser Asn Asn Thr Glu Gly

ATG GAC ACA ATC ACC CTC CCA TGC AGA ATA
Ser Asp Thr Ile Thr Leu Pro Cys Arg Ile

AAA CAA ATT ATA AAC ATG TGG CAG GAA GTA
Lys Gln Ile Ile Asn Met Trp Gln Glu Val

GGA AAA GCA ATG TAT GCC CCT CCC ATC AGT
Gly Lys Ala Met Tyr Ala Pro Pro Ile Ser

GGA CAA ATT AGA TGT TCA TCA AAT ATT ACA
Gly Gln Ile Arg Cys Ser Ser Asn Ile Thr

GGG CTG CTA TTA ACA AGA GAT GGT GGT AAT
Gly Leu Leu Leu Thr Arg Asp Gly Gly Asn

AGC AAC AAT GAG TCC GAG ATC TTC AGA CCT
Ser Asn Asn Glu Ser Glu Ile Phe Arg Pro

GGA GGA GGA GAT ATG AGG GAC AAT TGG AGA
Gly Gly Gly Asp Met Arg Asp Asn Trp Arg

AGT GAA TTA TAT AAA TAT AAA GTA GTA AAA
Ser Glu Leu Tyr Lys Tyr Lys Val Val Lys
 
ATT GAA CCA TTA GGA GTA GCA CCC ACC AAG
Ile Glu Pro Leu Gly Val Ala Pro Thr Lys

GCA AAG AGA AGA GTG GTG CAG AGA GAA AAA
Ala Lys Arg Arg Val Val Gln Arg Glu Lys

AGA|GCA GTG GGA ATA GGA CAG GCC AGA CAA
Arg|Ala Val Gly Ile Gly Gln Ala Arg Gln

TTA TTG TCT GGT ATA GTG CAG CAG CAG AAC
Leu Leu Ser Gly Ile Val Gln Gln Gln Asn

AAT TTG CTG AGG GCT ATT GAG GGC CAA CAG
Asn Leu Leu Arg Ala Ile Glu Gly Gln Gln

CAT CTG TTG CAA CTC ACA GTC TGG GGC ATC
His Leu Leu Gln Leu Thr Bal Trp Gly Ile

AAG CAG CTC CAG GCA AGA ATC CTG GCT GTG
Lys Gln Leu Gln Ala Arg Ile Leu Ala Val

GAA AGA TAC CTA AAG GAT CAA CAG CTC CTG
Glu Arg Tyr Leu Lys Asp Gln Gln Leu Leu

GGG ATT TGG GGT TGC TCT GGA AAA CTC ATT
Gly Ile Trp Gly Cys Ser Gly Lys Leu Ile

TGC ACC ACT GCT GTG CCT TGG AAT GCT AGT
Cys Thr Thr Ala Val Pro Trp Asn Ala Ser

TGG AGT AAT AAA TCT CTG GAA CAG ATT TGG
Trp Ser Asn Lys Ser Leu Glu Fln Ile Trp

AAT AAC ATG ACC TGG ATG GAG TGG GAC AGA
Asn Asn Met Thr Trp Met Flu Trp Asp Arg

GAA ATT AAC AAT TAC ACA AGC TTA ATA CAC
Glu Ile Asn Asn Tyr Thr Ser Leu Ile His

TCC TTA ATT GAA GAA TCG CAA AAC CAG CAA
Ser Leu Ile Glu Glu Ser Gln Asn Gln Gln

GAA AAG AAT GAA CAA GAA TTA
Glu Lys Asn Glu Gln Glu Leu

TTG GAA TTA GAT AAA TGG GCA
Leu Glu Leu Asp Lys Trp Ala
 the process comprising coupling amino acids by peptide bond formation.

5. A process for preparing an antibody, the process comprising using the peptide fragments as in any one of claims 1 to 4 as an immunogen, and optionally immortalising a cell producing the antibody.

6. A method for the detection of antibodies against the HTLV-III virus comprising contacting a peptide fragment produceable by a process as claimed in any one of claims 1 to 4 with a sample suspected of containing antibodies against the HTLV-III virus and detecting the presence of sad antibodies.

7. A method for the detection of the HTLV-III virus or portions thereof comprising contacting a sample suspected of containing the HTLV-III virus or portions thereof with an antibody produceable by a process as claimed in claim 5 and detecting the presence of said virus.

8. A process for the preparation of an expression vehicle comprising a polynucleotide sequence coding for a peptide fragment produceable by a process as claimed in any one of claims 1 to 4, the process comprising joining together polynucleotides and/or successive nucleotides.

9. A process as claimed in claim 8, wherein the expression vehicle is a plasmid.

10. A process as claimed in claim 9, wherein the plasmid is selected from pJLB0T, pJLB1T, and pJLB2T (as described in Example 1 and depicted in Figure 2); pJLB0TR, pJLB1TR, and pJLB2TR (as described in Example 1 and depicted in Figure 3); and pLCBC0, pLCBC1, pLCBC2, pLCBC00, pLCBC10, pLCBC20 (as described in Example 2 and depicted in Figure 10).

11. A process for the preparation of a host cell the process comprising transforming a cell with the expression vehicle comprising a polynucleotide sequence coding for a peptide fragment produceable by a process as claimed in any of claims 1 to 4.

12. A method of expressing a peptide fragment produceable by a process as in any of claims 1 to 4 comprising transforming a host cell with an expression vehicle comprising a polynucleotide sequence coding for said peptide fragment, culturing said transformed host, and expressing said peptide fragment.

13. A process for the production of a vaccine against the HTLV-III virus comprising admixing an effective amount of a peptide fragment produceable by a process as claimed in any of claims 1 to 4 with an acceptable carrier.

14. The use of a peptide fragment produceable by a process as claimed in any one of claims 1 to 4 in the preparation of a vaccine against the HTLV-III virus.

15. A kit for detecting HTLV-III antibodies comprising a carrier means compartmentalized to receive one or more container means, at least one of said container means containing an immobilized peptide fragment produceable by a process as claimed in any one of claims 1 to 4.

16. A kit for detecting the HTLV-III virus or portions thereof comprising a carrier means compartmentalized to receive one or more container means, at least one of said one or more container means containing an antibody produceable by a process as claimed in claim 5.

## Patentansprüche (Patentansprüche für folgende(n) Vertragsstaat(en): BE, FR, DE, IT, GR, NL, SE, CH, LI, LU, GB)

1. Peptidfragment, welches auf Antikörper gegen das HTLV-III-Virus immunreaktiv und durch die HTLV-III-Provirus-Nukleotidsequenz 7199 bis 8052 kodiert ist.

2. Peptidfragment, welches auf Antikörper gegen das HTLV-III-Virus immunreaktiv und durch eine Nukleotidsequenz des HTLV-III-Provirus, zumindest von ungefähr 7199 bis ungefähr 7810, kodiert ist, welches die hydrophoben Bereiche 7350 bis 7418 sowie 7851 bis 7916 ausschließt, und welches ein dem Aminoende vorgelagertes Segment bis ungefähr zum Nukleotid 6827 enthält.

3. Peptidfragment, welches auf Antikörper gegen das HTLV-III-Virus immunreaktiv und durch eine Nukleotidsequenz des HTLV-III-Provirus von ungefähr 6827-6936, 6952-7349, 7419-7802, unter Ausschluß von 6937-6951 sowie 7350-7418, kodiert ist.

4. Peptidsequenz, umfassend die Aminosäurereste, welche in der unteren der beiden Zeilen im folgenden Diagramm wiedergegeben werden:

AAA CAG ATA
Lys Gln Ile

GCT AGC AAA TTA AGA GAA CAA TTT GGA AAT
Ala Ser Lys Leu Arg Glu Gln Phe Gly Asn

AAT AAA ACA ATA ATC TTT AAG CAG TCC TCA
Asn Lys Thr Ile Ile Phe Lys Gln Ser Ser

GGA GGG GAC CCA GAA ATT GTA ACG CAC AGT
Gly Gly Asp Pro Glu Ile Bal Thr His Ser

TTT AAT TGT GGA GGG GAA TTT TTC TAC TGT
Phe Asn Cys Gly Gly Glu Phe Phe Tyr Cys

AAT TCA ACA CAA CTG TTT AAT AGT ACT TGG
Asn Ser Thr Gln Leu Phe Asn Ser Thr Trp

AGT ACT AAA GGG TCA AAT AAC ACT GAA GGA
Ser Thr Lys Gly Ser Asn Asn Thr Glu Gly

ATG GAC ACA ATC ACC CTC CCA TGC AGA ATA
Ser Asp Thr Ile Thr Leu Pro Cys Arg Ile

AAA CAA ATT ATA AAC ATG TGG CAG GAA GTA
Lys Gln Ile Ile Asn Met Trp Gln Glu Val

GGA AAA GCA ATG TAT GCC CCT CCC ATC AGT
Gly Lys Ala Met Tyr Ala Pro Pro Ile Ser

GGA CAA ATT AGA TGT TCA TCA AAT ATT ACA
Gly Gln Ile Arg Cys Ser Ser Asn Ile Thr

GGG CTG CTA TTA ACA AGA GAT GGT GGT AAT
Gly Leu Leu Leu Thr Arg Asp Gly Gly Asn

AGC AAC AAT GAG TCC GAG ATC TTC AGA CCT
Ser Asn Asn Glu Ser Glu Ile Phe Arg Pro

GGA GGA GGA GAT ATG AGG GAC AAT TGG AGA
Gly Gly Gly Asp Met Arg Asp Asn Trp Arg

AGT GAA TTA TAT AAA TAT AAA GTA GTA AAA
Ser Glu Leu Tyr Lys Tyr Lys Val Val Lys

ATT GAA CCA TTA GGA GTA GCA CCC ACC AAG
Ile Glu Pro Leu Gly Val Ala Pro Thr Lys

GCA AAG AGA AGA GTG GTG CAG AGA GAA AAA
Ala Lys Arg Arg Val Val Gln Arg Glu Lys

AGA|GCA GTG GGA ATA GGA CAG GCC AGA CAA
Arg|Ala Val Gly Ile Gly Gln Ala Arg Gln

TTA TTG TCT GGT ATA GTG CAG CAG CAG AAC
Leu Leu Ser Gly Ile Val Gln Gln Gln Asn

AAT TTG CTG AGG GCT ATT GAG GGC CAA CAG
Asn Leu Leu Arg Ala Ile Glu Gly Gln Gln

CAT CTG TTG CAA CTC ACA GTC TGG GGC ATC
His Leu Leu Gln Leu Thr Bal Trp Gly Ile

AAG CAG CTC CAG GCA AGA ATC CTG GCT GTG
Lys Gln Leu Gln Ala Arg Ile Leu Ala Val

GAA AGA TAC CTA AAG GAT CAA CAG CTC CTG
Glu Arg Tyr Leu Lys Asp Gln Gln Leu Leu

GGG ATT TGG GGT TGC TCT GGA AAA CTC ATT
Gly Ile Trp Gly Cys Ser Gly Lys Leu Ile

TGC ACC ACT GCT GTG CCT TGG AAT GCT AGT
Cys Thr Thr Ala Val Pro Trp Asn Ala Ser

TGG AGT AAT AAA TCT CTG GAA CAG ATT TGG
Trp Ser Asn Lys Ser Leu Glu Fln Ile Trp

AAT AAC ATG ACC TGG ATG GAG TGG GAC AGA
Asn Asn Met Thr Trp Met Flu Trp Asp Arg

GAA ATT AAC AAT TAC ACA AGC TTA ATA CAC
Glu Ile Asn Asn Tyr Thr Ser Leu Ile His

TCC TTA ATT GAA GAA TCG CAA AAC CAG CAA
Ser Leu Ile Glu Glu Ser Gln Asn Gln Gln

GAA AAG AAT GAA CAA GAA TTA
Glu Lys Asn Glu Gln Glu Leu

TTG GAA TTA GAT AAA TGG GCA
Leu Glu Leu Asp Lys Trp Ala

5. Antikörper, welcher durch Verwendung eines Peptidfragmentes nach einem der Ansprüche 1 bis 4 als Immunogen gewonnen wird.

6. Verfahren zum Feststellen von Antikörpern gegen das HTLV-III-Virus_{,} bei welchem ein Peptidfragment nach einem der Ansprüche 1 bis 4 mit einer Probe, von welcher vermutet wird, daß sie Antikörper gegen das HTLV-III-Virus enthält, zusammengebracht und das Vorhandensein dieser Antikörper festgestellt wird.

7. Verfahren zum Feststellen des HTLV-III-Virus oder von Teilen davon, bei welchem eine Probe, von welcher vermutet wird, daß sie das HTLV-III-Virus oder Teile davon enthält, mit einem Antikörper aus Anspruch 5 zusammengebracht und das Vorhandensein dieses Virus festgestellt wird.

8. Expressionsvehikel, welche eine Polynukleotidsequenz enthält, die für ein Peptidfragment nach einem der Ansprüche 1 bis 4 kodiert.

9. Expressionsvehikel nach Anspruch 8, welches ein Plasmid ist.

10. Plasmid nach Anspruch 9, welches ist: pJLB0T, pJLB1T und pJLB2T (wie in Beispiel 1 beschrieben und in den Figuren 1 und 2 dargestellt wird); pJLB0TR, pJLB1TR und pJLB2TR (wie in Beispiel 1 beschrieben und in Figur 3 dargestellt wird); sowie pLCBC0, pLCBC1, pLCBC2, pLCBC00, pLCBC10, pLCBC20 (wie in Beispiel 2 beschrieben und in den Figuren 4 und 10 dargestellt wird).

11. Wirtszelle, welche mit einem Expressionsvehikel nach einem der Ansprüche 8 bis 10 transformiert wird.

12. Verfahren zum Exprimieren eines Peptidfragments nach einem der Ansprüche 1 bis 4, bei welchem eine Wirtszelle mit einem Expressionsvehikel, welches eine für dieses Peptidfragment kodierende Polynukleotidsequenz umfaßt, transformiert, eine Kultur des transformierten Wirtes angelegt und das Peptidfragment exprimiert wird.

13. Impfstoff gegen das HTLV-III-Virus, welcher eine wirksame Menge eines Peptidfragments nach einem der Ansprüche 1 bis 4 und einen zulässigen Träger enthält.

14. Verwendung eines Peptidfragments nach einem der Ansprüche 1 bis 4 bei der Herstellung eines Impfstoffes gegen das HTLV-III-Virus.

15. Ausrüstungssatz zum Feststellen von HTLV-III-Antikörpern, welcher einen zur Aufnahme eines oder mehrerer Behälter in Fächer geteilten Träger aufweist, wobei zumindest ein der Behälter ein immobilisiertes Peptidfragment nach einem der Ansprüche 1 bis 4 enthält.

16. Ausrüstungssatz zum Feststellen des HTLV-III-Virus oder Teilen davon, welcher einen zur Aufnahme eines oder mehrerer Behälter in Fächer geteilten Träger aufweist, wobei zumindest einer des einen oder der mehreren Behälter einen Antikörper nach Anspruch 5 enthält.

## Patentansprüche (Patentansprüche für folgende(n) Vertragsstaat(en): AT, ES)

1. Verfahren zum Herstellen eines Peptidfragments, welches auf Antikörper gegen das HTLV-III-Virus immunreaktiv und durch die HLTV-III-Provirus-Nukleotidsequenz 7199 bis 8052 kodiert ist, wobei das Verfahren das Koppeln von Aminosäuren durch Bildung von Peptidbindungen umfaßt.

2. Verfahren zum Herstellen eines Peptidfragments, welches auf Antikörper gegen das HTLV-III-Virus immunreaktiv und durch eine Nukleotidsequenz des HTLV-III-Provirus, zumindest von ungefähr 7199 bis ungefähr 7810, kodiert ist, welches die hydrophoben Bereiche 7350 bis 7418 sowie 7851 bis 7916 ausschließt, und welches ein dem Aminoende vorgelagertes Segment bis ungefähr zum Nukleotid 6827 enthält, wobei das Verfahren das Koppeln von Aminosäuren durch Bildung von Peptidbindungen umfaßt.

3. Verfahren zum Herstellen eines Peptidfragments, welches auf Antikörper gegen das HTLV-III-Virus immunreaktiv und durch eine Nukleotidsequenz des HTLV-III-Provirus von ungefähr 6827-6936, 6952-7349, 7419-7802, unter Ausschluß von 6937-6951 sowie 7350-7418, kodiert ist, wobei das Verfahren das Koppeln von Aminosäuren durch Bildung von Peptidbindungen umfaßt.

4. Verfahren zum Herstellen einer Peptidsequenz, umfassend die Aminosäurereste, welche in der unteren der beiden Zeilen im folgenden Diagramm wiedergegeben werden: 
AAA CAG ATA
Lys Gln Ile

GCT AGC AAA TTA AGA GAA CAA TTT GGA AAT
Ala Ser Lys Leu Arg Glu Gln Phe Gly Asn

AAT AAA ACA ATA ATC TTT AAG CAG TCC TCA
Asn Lys Thr Ile Ile Phe Lys Gln Ser Ser

GGA GGG GAC CCA GAA ATT GTA ACG CAC AGT
Gly Gly Asp Pro Glu Ile Bal Thr His Ser
 
TTT AAT TGT GGA GGG GAA TTT TTC TAC TGT
Phe Asn Cys Gly Gly Glu Phe Phe Tyr Cys

AAT TCA ACA CAA CTG TTT AAT AGT ACT TGG
Asn Ser Thr Gln Leu Phe Asn Ser Thr Trp

AGT ACT AAA GGG TCA AAT AAC ACT GAA GGA
Ser Thr Lys Gly Ser Asn Asn Thr Glu Gly

ATG GAC ACA ATC ACC CTC CCA TGC AGA ATA
Ser Asp Thr Ile Thr Leu Pro Cys Arg Ile

AAA CAA ATT ATA AAC ATG TGG CAG GAA GTA
Lys Gln Ile Ile Asn Met Trp Gln Glu Val

GGA AAA GCA ATG TAT GCC CCT CCC ATC AGT
Gly Lys Ala Met Tyr Ala Pro Pro Ile Ser

GGA CAA ATT AGA TGT TCA TCA AAT ATT ACA
Gly Gln Ile Arg Cys Ser Ser Asn Ile Thr

GGG CTG CTA TTA ACA AGA GAT GGT GGT AAT
Gly Leu Leu Leu Thr Arg Asp Gly Gly Asn

AGC AAC AAT GAG TCC GAG ATC TTC AGA CCT
Ser Asn Asn Glu Ser Glu Ile Phe Arg Pro

GGA GGA GGA GAT ATG AGG GAC AAT TGG AGA
Gly Gly Gly Asp Met Arg Asp Asn Trp Arg

AGT GAA TTA TAT AAA TAT AAA GTA GTA AAA
Ser Glu Leu Tyr Lys Tyr Lys Val Val Lys

ATT GAA CCA TTA GGA GTA GCA CCC ACC AAG
Ile Glu Pro Leu Gly Val Ala Pro Thr Lys

GCA AAG AGA AGA GTG GTG CAG AGA GAA AAA
Ala Lys Arg Arg Val Val Gln Arg Glu Lys

AGA|GCA GTG GGA ATA GGA CAG GCC AGA CAA
Arg|Ala Val Gly Ile Gly Gln Ala Arg Gln

TTA TTG TCT GGT ATA GTG CAG CAG CAG AAC
Leu Leu Ser Gly Ile Val Gln Gln Gln Asn

AAT TTG CTG AGG GCT ATT GAG GGC CAA CAG
Asn Leu Leu Arg Ala Ile Glu Gly Gln Gln

CAT CTG TTG CAA CTC ACA GTC TGG GGC ATC
His Leu Leu Gln Leu Thr Bal Trp Gly Ile

AAG CAG CTC CAG GCA AGA ATC CTG GCT GTG
Lys Gln Leu Gln Ala Arg Ile Leu Ala Val
 
GAA AGA TAC CTA AAG GAT CAA CAG CTC CTG
Glu Arg Tyr Leu Lys Asp Gln Gln Leu Leu

GGG ATT TGG GGT TGC TCT GGA AAA CTC ATT
Gly Ile Trp Gly Cys Ser Gly Lys Leu Ile

TGC ACC ACT GCT GTG CCT TGG AAT GCT AGT
Cys Thr Thr Ala Val Pro Trp Asn Ala Ser

TGG AGT AAT AAA TCT CTG GAA CAG ATT TGG
Trp Ser Asn Lys Ser Leu Glu Fln Ile Trp

AAT AAC ATG ACC TGG ATG GAG TGG GAC AGA
Asn Asn Met Thr Trp Met Flu Trp Asp Arg

GAA ATT AAC AAT TAC ACA AGC TTA ATA CAC
Glu Ile Asn Asn Tyr Thr Ser Leu Ile His

TCC TTA ATT GAA GAA TCG CAA AAC CAG CAA
Ser Leu Ile Glu Glu Ser Gln Asn Gln Gln

GAA AAG AAT GAA CAA GAA TTA
Glu Lys Asn Glu Gln Glu Leu

TTG GAA TTA GAT AAA TGG GCA
Leu Glu Leu Asp Lys Trp Ala
 wobei das Verfahren das Koppeln von Aminosäuren durch Bildung von Peptidbindungen umfaßt.

5. Verfahren zum Herstellen eines Antikörpers, wobei das Verfahren die Verwendung des Peptidfragmentes nach einem der Ansprüche 1 bis 4 als Immunogen und gegebenenfalls das Immortalisieren einer Zelle, welche den Antikörper erzeugt, umfaßt.

6. Verfahren zum Feststellen von Antikörpern gegen das HTLV-III-Virus, bei welchem ein durch ein Verfahren nach einem der Ansprüche 1 bis 4 herstellbares Peptidfragment mit einer Probe, von welcher vermutet wird, daß sie Antikörper gegen das HTLV-III-Virus enthält, zusammengebracht und das Vorhandenseins dieser Antikörper festgestellt wird.

7. Verfahren zum Feststellen des HTLV-III-Virus oder von Teilen davon, bei welchem eine Probe, von welcher vermutet wird, daß sie das HTLV-III-Virus oder Teile davon enthält, mit einem durch ein Verfahren nach Anspruch 5 herstellbaren Antikörper zusammengebracht und das Vorhandensein dieses Virus festgestellt wird..

8. Verfahren zum Herstellen eines Expressionsvehikels, das eine für ein durch ein Verfahren nach einem der Ansprüche 1 bis 4 herstellbares Peptidfragment kodierende Polynucleotidsequenz umfaßt, wobei das Verfahren das Verbinden von Polynukleotiden und/oder aufeinanderfolgenden Nukleotiden umfaßt.

9. Verfahren nach Anspruch 8, wobei das Expressionsvehikel ein Plasmid ist.

10. Verfahren nach Anspruch 9, wobei das Plasmid ausgewählt wird aus pJLB0T, pJLB1T und pJLB2T (wie in Beispiel 1 beschrieben und in den Figuren 1 und 2 dargestellt wird); pJLB0TR, pJLB1TR und pJLB2TR (wie in Beispiel 1 beschrieben und in Figur 3 dargestellt wird); sowie pLCBC0, pLCBC1, pLCBC2, pLCBC00, pLCBC10, pLCBC20 (wie in Beispiel 2 beschrieben und in den Figuren 4 und 10 dargestellt wird).

11. Verfahren zum Herstellen einer Wirtzelle, wobei das Verfahren das Transformieren einer Zelle mit dem Expressionsvehikel umfaßt, das eine für ein durch ein Verfahren nach einem der Ansprüche 1 bis 4 herstellbares Peptidfragment kodierende Polynukleotidsequenz aufweist.

12. Verfahren zum Exprimieren eines durch ein Verfahren nach einem der Ansprüche 1 bis 4 herstellbaren Peptidfragments, wobei eine Wirtszelle mit einem Expressionsvehikel, das eine für das Peptidfragment kodierende Polynukleotidsequenz aufweist, transformiert, eine Kultur des transformierten Wirtes angelegt und das Peptidfragment exprimiert wird.

13. Verfahren zum Herstellen eines Impfstoffes gegen das HTLV-III-Virus, bei welchem eine wirksame Menge eines durch ein Verfahren nach einem der Ansprüche 1 bis 4 herstellbaren Peptidfragments einem zulässigen Träger beigemischt wird.

14. Verwendung eines durch ein Verfahren nach einem der Ansprüche 1 bis 4 herstellbaren Peptidfragments bei der Herstellung eines Impfstoffes gegen das HTLV-III-Virus.

15. Ausrüstungssatz zum Feststellen HTLV-III-Antikörpern, welcher einen zur Aufnahme eines oder mehrerer Behälter in Fächer geteilten Träger aufweist, wobei zumindest einer der Behälter ein immobilisiertes durch ein Verfahren nach einem der Ansprüche 1 bis 4 herstellbares Peptidfragment enthält.

16. Ausrüstungssatz zum Feststellen des HTLV-III-Virus oder Teilen davon, welcher einen zur Aufnahme eines oder mehrerer Behälter in Fächer geteilten Träger aufweist, wobei zumindest einer des einen oder der mehreren Behälter einen durch ein Verfahren nach Anspruch 5 herstellbaren Antikörper enthält.

## Revendications (Revendications pour l'(les) Etat(s) contractant(s) suivant(s): BE, FR, DE, IT, GR, NL, SE, CH, LI, LU, GB)

1. Fragment peptidique qui est immunoréactif vis-à-vis d'anticorps contre le virus HTLV-III et qui est codé par la séquence nucléotidique 7199 à 8052 du provirus HTLV-III.

2. Fragment peptidique qui est immunoréactif vis-à-vis d'anticorps contre le virus HTLV-III et qui est codé par une séquence nucléotidique du provirus HTLV-III d'au moins d'environ 7199 à environ 7810, qui exclut les régions hydrophobes 7350 à 7418 et 7851 à 7916 et qui inclut un segment en amont de l'extrémité amino, à environ le nucléotide 6827.

3. Fragment peptidique qui est immunoréactif vis-à-vis d'anticorps contre le virus HTLV-III et qui est codé par une séquence nucléotidique du provirus HTLV-III, d'environ 6827-6936, 6952-7349, 7419-7802, excluant 6937-6951 et 7350-7418.

4. Séquence peptidique comprenant les résidus acide aminé donnés à la ligne inférieure du diagramme à deux lignes suivant :

AAA CAG ATA
Lys Gln Ile

GCT AGC AAA TTA AGA GAA CAA TTT GGA AAT
Ala Ser Lys Leu Arg Glu Gln Phe Gly Asn

AAT AAA ACA ATA ATC TTT AAG CAG TCC TCA
Asn Lys Thr Ile Ile Phe Lys Gln Ser Ser

GGA GGG GAC CCA GAA ATT GTA ACG CAC AGT
Gly Gly Asp Pro Glu Ile Bal Thr His Ser

TTT AAT TGT GGA GGG GAA TTT TTC TAC TGT
Phe Asn Cys Gly Gly Glu Phe Phe Tyr Cys

AAT TCA ACA CAA CTG TTT AAT AGT ACT TGG
Asn Ser Thr Gln Leu Phe Asn Ser Thr Trp

AGT ACT AAA GGG TCA AAT AAC ACT GAA GGA
Ser Thr Lys Gly Ser Asn Asn Thr Glu Gly

ATG GAC ACA ATC ACC CTC CCA TGC AGA ATA
Ser Asp Thr Ile Thr Leu Pro Cys Arg Ile

AAA CAA ATT ATA AAC ATG TGG CAG GAA GTA
Lys Gln Ile Ile Asn Met Trp Gln Glu Val

GGA AAA GCA ATG TAT GCC CCT CCC ATC AGT
Gly Lys Ala Met Tyr Ala Pro Pro Ile Ser

GGA CAA ATT AGA TGT TCA TCA AAT ATT ACA
Gly Gln Ile Arg Cys Ser Ser Asn Ile Thr

GGG CTG CTA TTA ACA AGA GAT GGT GGT AAT
Gly Leu Leu Leu Thr Arg Asp Gly Gly Asn

AGC AAC AAT GAG TCC GAG ATC TTC AGA CCT
Ser Asn Asn Glu Ser Glu Ile Phe Arg Pro

GGA GGA GGA GAT ATG AGG GAC AAT TGG AGA
Gly Gly Gly Asp Met Arg Asp Asn Trp Arg

AGT GAA TTA TAT AAA TAT AAA GTA GTA AAA
Ser Glu Leu Tyr Lys Tyr Lys Val Val Lys

ATT GAA CCA TTA GGA GTA GCA CCC ACC AAG
Ile Glu Pro Leu Gly Val Ala Pro Thr Lys

GCA AAG AGA AGA GTG GTG CAG AGA GAA AAA
Ala Lys Arg Arg Val Val Gln Arg Glu Lys

AGA|GCA GTG GGA ATA GGA CAG GCC AGA CAA
Arg|Ala Val Gly Ile Gly Gln Ala Arg Gln

TTA TTG TCT GGT ATA GTG CAG CAG CAG AAC
Leu Leu Ser Gly Ile Val Gln Gln Gln Asn

AAT TTG CTG AGG GCT ATT CAG GGC CAA CAG
Asn Leu Leu Arg Ala Ile Glu Gly Gln Gln

CAT CTG TTG CAA CTC ACA GTC TGG GGC ATC
His Leu Leu Gln Leu Thr Bal Trp Gly Ile

AAG CAG CTC CAG GCA AGA ATC CTG GCT GTG
Lys Gln Leu Gln Ala Arg Ile Leu Ala Val

GAA AGA TAC CTA AAG GAT CAA CAG CTC CTG
Glu Arg Tyr Leu Lys Asp Gln Gln Leu Leu

GGG ATT TGG GGT TGC TCT GGA AAA CTC ATT
Gly Ile Trp Gly Cys Ser Gly Lys Leu Ile

TGC ACC ACT GCT GTG CCT TGG AAT GCT AGT
Cys Thr Thr Ala Val Pro Trp Asn Ala Ser

TGG AGT AAT AAA TCT CTG GAA CAG ATT TGG
Trp Ser Asn Lys Ser Leu Glu Fln Ile Trp

AAT AAC ATG ACC TGG ATG GAG TGG GAC AGA
Asn Asn Met Thr Trp Met Flu Trp Asp Arg

GAA ATT ACC AAT TAC ACA AGC TTA ATA CAC
Glu Ile Asn Asn Tyr Thr Ser Leu Ile His

TCC TTA ATT GAA GAA TCG CAA AAC CAG CAA
Ser Leu Ile Glu Glu Ser Gln Asn Gln Gln

GAA AAG AAT GAA CAA GAA TTA
Glu Lys Asn Glu Gln Glu Leu

TTG GAA TTA GAT AAA TGG GCA
Leu Glu Leu Asp Lys Trp Ala

5. Anticorps obtenu au moyen d'un fragment peptidique suivant l'une quelconque des revendications 1 à 4, comme immunogène.

6. Procédé de détection d'anticorps contre le virus HTLV-III, comprenant la mise en contact d'un fragment peptidique suivant l'une quelconque des revendications 1 à 4, avec un échantillon suspecté contenir les anticorps contre le virus HTLV-III et la détection de la présence des anticorps.

7. Procédé de détection du virus HTLV-III ou de fractions de celui-ci, comprenant la mise en contact d'un échantillon suspecté contenir le virus HTLV-III ou des fractions de celui-ci avec un anticorps suivant la revendication 5 et la détection de la présence du virus.

8. Véhicule d'expression comprenant une séquence polynucléotidique codant pour un fragment peptidique suivant l'une quelconque des revendications 1 à 4.

9. Véhicule d'expression suivant la revendication 8, qui est un plasmide.

10. Plasmide suivant la revendication 9, qui est :
le pJLB0T, le pJLB1T et le pJLB2T (comme décrit à l'exemple 1 et représenté aux Fig. 1 et 2);
le pJLB0TR, le pJLB1TR et le pJLB2TR (comme décrit à l'exemple l et représenté à la Fig. 3), et
le pLCBC0, le pLCBC1, le pLCBC2, le pLCBC00, le pLCBC10, le pLCBC20 (comme décrit à l'exemple 2 et représenté aux Fig. 4 à 10).

11. Cellule hôte transformée par un véhicule d'expression suivant l'une quelconque des revendications 8 à 10.

12. Procédé d'expression d'un fragment peptidique suivant l'une quelconque des revendications 1 à 4, comprenant la transformation d'une cellule hôte avec un véhicule d'expression comprenant une séquence polynucléotidique codant pour le fragment peptidique, la mise en culture de l'hôte transformé et l'expression du fragment peptidique.

13. Vaccin contre le virus HTLV-III comprenant une quantité efficace d'un fragment peptidique suivant l'une quelconque des revendications 1 à 4 et un excipient acceptable.

14. Utilisation d'un fragment peptidique suivant l'une quelconque des revendications 1 à 4, dans la préparation d'un vaccin contre le virus HTLV-III.

15. Equipement pour la détection d'anticorps HTLV-III, comprenant un dispositif support compartimenté pour recevoir un ou plusieurs dispositifs récepteurs, au moins l'un de ces dispositifs récepteurs contenant un fragment peptidique immobilisé suivant l'une quelconque des revendications 1 à 4.

16. Equipement pour la détection du virus HTLV-III ou des fractions de celui-ci, comprenant un dispositif support compartimenté pour recevoir un ou plusieurs dispositifs récepteurs, au moins l'un de ces dispositifs récepteurs contenant un anticorps suivant la revendication 5.

## Revendications (Revendications pour l'(les) Etat(s) contractant(s) suivant(s): AT, ES)

1. Procédé de préparation d'un fragment peptidique qui est immunoréactif vis-à-vis d'anticorps contre le virus HTLV-III et qui est codé par la séquence nucléotidique 7199 à 8052 du provirus HTLV-III, le procédé comprenant le couplage d'acides aminés par formation d'un lien peptidique.

2. Procédé de préparation d'un fragment peptidique qui est immunoréactif vis-à-vis d'anticorps contre le virus HTLV-III et qui est codé par une séquence nucléotidique du provirus HTLV-III, d'au moins d'environ 7199 à environ 7810, qui exclut les régions hydrophobes 7350 à 7418 et 7851 à 7916 et qui inclut un segment en amont de l'extrémité amino, à environ le nucléotide 6827, le procédé comprenant le couplage d'acides aminés par formation d'un lien peptidique.

3. Procédé de préparation d'un fragment peptidique qui est immunoréactif vis-à-vis d'anticorps contre le virus HTLV-III et qui est codé par une séquence nucléotidique du provirus HTLV-III, d'environ 6827-6936, 6952-7349, 7419-7802, excluant 6937-6951 et 7350-7418, le procédé comprenant le couplage d'acides aminés par formation d'un lien peptidique.

4. Procédé de préparation d'une séquence peptidique comprenant les résidus acide aminé donnés à la ligne inférieure du diagramme à deux lignes suivant : 
AAA CAG ATA
Lys Gln Ile

GCT AGC AAA TTA AGA GAA CAA TTT GGA AAT
Ala Ser Lys Leu Arg Glu Gln Phe Gly Asn

AAT AAA ACA ATA ATC TTT AAG CAG TCC TCA
Asn Lys Thr Ile Ile Phe Lys Gln Ser Ser

GGA GGG GAC CCA GAA ATT GTA ACG CAC AGT
Gly Gly Asp Pro Glu Ile Bal Thr His Ser
 
TTT AAT TGT GGA GGG GAA TTT TTC TAC TGT
Phe Asn Cys Gly Gly Glu Phe Phe Tyr Cys

AAT TCA ACA CAA CTG TTT AAT AGT ACT TGG
Asn Ser Thr Gln Leu Phe Asn Ser Thr Trp

AGT ACT AAA GGG TCA AAT AAC ACT GAA GGA
Ser Thr Lys Gly Ser Asn Asn Thr Glu Gly

ATG GAC ACA ATC ACC CTC CCA TGC AGA ATA
Ser Asp Thr Ile Thr Leu Pro Cys Arg Ile

AAA CAA ATT ATA AAC ATG TGG CAG GAA GTA
Lys Gln Ile Ile Asn Met Trp Gln Glu Val

GGA AAA GCA ATG TAT GCC CCT CCC ATC AGT
Gly Lys Ala Met Tyr Ala Pro Pro Ile Ser

GGA CAA ATT AGA TGT TCA TCA AAT ATT ACA
Gly Gln Ile Arg Cys Ser Ser Asn Ile Thr

GGG CTG CTA TTA ACA AGA GAT GGT GGT AAT
Gly Leu Leu Leu Thr Arg Asp Gly Gly Asn

AGC AAC AAT GAG TCC GAG ATC TTC AGA CCT
Ser Asn Asn Glu Ser Glu Ile Phe Arg Pro

GGA GGA GGA GAT ATG AGG GAC AAT TGG AGA
Gly Gly Gly Asp Met Arg Asp Asn Trp Arg

AGT GAA TTA TAT AAA TAT AAA GTA GTA AAA
Ser Glu Leu Tyr Lys Tyr Lys Val Val Lys

ATT GAA CCA TTA GGA GTA GCA CCC ACC AAG
Ile Glu Pro Leu Gly Val Ala Pro Thr Lys

GCA AAG AGA AGA GTG GTG CAG AGA GAA AAA
Ala Lys Arg Arg Val Val Gln Arg Glu Lys

AGA|GCA GTG GGA ATA GGA CAG GCC AGA CAA
Arg|Ala Val Gly Ile Gly Gln Ala Arg Gln

TTA TTG TCT GGT ATA GTG CAG CAG CAG AAC
Leu Leu Ser Gly Ile Val Gln Gln Gln Asn

AAT TTG CTG AGG GCT ATT CAG GGC CAA CAG
Asn Leu Leu Arg Ala Ile Glu Gly Gln Gln

CAT CTG TTG CAA CTC ACA GTC TGG GGC ATC
His Leu Leu Gln Leu Thr Bal Trp Gly Ile

AAG CAG CTC CAG GCA AGA ATC CTG GCT GTG
Lys Gln Leu gln Ala Arg Ile Leu Ala Val
 
GAA AGA TAC CTA AAG GAT CAA CAG CTC CTG
Glu Arg Tyr Leu Lys Asp Gln Gln Leu Leu

GGG ATT TGG GGT TGC TCT GGA AAA CTC ATT
Gly Ile Trp Gly Cys Ser Gly Lys Leu Ile

TGC ACC ACT GCT GTG CCT TGG AAT GCT AGT
Cys Thr Thr Ala Val Pro Trp Asn Ala Ser

TGG AGT AAT AAA TCT CTG GAA CAG ATT TGG
Trp Ser Asn Lys Ser Leu Glu Fln Ile Trp

AAT AAC ATG ACC TGG ATG GAG TGG GAC AGA
Asn Asn Met Thr Trp Met Flu Trp Asp Arg

GAA ATT ACC AAT TAC ACA AGC TTA ATA CAC
Glu Ile Asn Asn Tyr Thr Ser Leu Ile His

TCC TTA ATT GAA GAA TCG CAA AAC CAG CAA
Ser Leu Ile Glu Glu Ser Gln Asn Gln Gln

GAA AAG AAT GAA CAA GAA TTA
Glu Lys Asn Glu Gln Glu Leu

TTG GAA TTA GAT AAA TGG GCA
Leu Glu Leu Asp Lys Trp Ala
 le procédé comprenant le couplage d'acides aminés par formation d'un lien peptidique.

5. Procédé de préparation d'un anticorps, le procédé comprenant l'utilisation des fragments peptidiques suivant l'une quelconque des revendications 1 à 4 comme immunogène et facultativement, l'immortalisation d'une cellule produisant l'anticorps.

6. Procédé de détection d'anticorps contre le virus HTLV-III, comprenant la mise en contact d'un fragment peptidique pouvant être obtenu par un procédé suivant l'une quelconque des revendications 1 à 4, avec un échantillon suspecté contenir des anticorps contre le virus HTLV-III et la détection de la présence des anticorps.

7. Procédé de détection du virus HTLV-III ou de fractions de celui-ci, comprenant la mise en contact d'un échantillon suspecté contenir le virus HTLV-III ou des fractions de celui-ci avec un anticorps pouvant être obtenu par un procédé suivant la revendication 5 et la détection de la présence du virus.

8. Procédé de préparation d'un véhicule d'expression comprenant une séquence polynucléotidique codant pour un fragment peptidique pouvant être obtenu par un procédé suivant l'une quelconque des revendications 1 à 4, le procédé comprenant la jonction de polynucléotides et/ou de nucléotides successifs.

9. Procédé suivant la revendication 8, dans lequel le véhicule d'expression est un plasmide.

10. Procédé suivant la revendication 9, dans lequel le plasmide est :
le pJLB0T, le pJLB1T et le pJLB2T (comme décrit à l'exemple l et représenté aux Fig. 1 et 2);
le pJLB0TR, le pJLB1TR et le pJLB2TR (comme décrit à l'exemple 1 et représenté à la Fig. 3), et
le pLCBC0, le pLCBC1, le pLCBC2, le pLCBC00, le pLCBC10, le pLCBC20 (comme décrit à l'exemple 2 et représenté à la Fig. 10).

11. Procédé de préparation d'une cellule hôte, le procédé comprenant la transformation d'une cellule par le véhicule d'expression comprenant une séquence polynucléotidique codant un fragment peptidique pouvant être obtenu par un procédé suivant l'une quelconque des revendications 1 à 4.

12. Procédé d'expression d'un fragment peptidique pouvant être obtenu par un procédé suivant l'une quelconque des revendications 1 à 4, comprenant la transformation d'une cellule hôte avec un véhicule d'expression comprenant une séquence polynucléotidique codant pour le fragment peptidique, la mise en culture de l'hôte transformé et l'expression du fragment peptidique.

13. Procédé de production d'un vaccin contre le virus HTLV-III comprenant le mélange d'une quantité efficace d'un fragment peptidique pouvant être obtenu suivant l'une quelconque des revendications 1 à 4, avec un excipient acceptable.

14. Utilisation d'un fragment peptidique pouvant être obtenu par un procédé suivant l'une quelconque des revendications 1 à 4, dans la préparation d'un vaccin contre le virus HTLV-III.

15. Equipement pour la détection d'anticorps HTLV-III, comprenant un dispositif support compartimenté pour recevoir un ou plusieurs dispositifs récepteurs, au moins l'un de ces dispositifs récepteurs contenant un fragment peptidique immobilisé pouvant être obtenu par un procédé suivant l'une quelconque des revendications 1 à 4.

16. Equipement pour la détection du virus HTLV-III ou des fractions de celui-ci, comprenant un dispositif support compartimenté pour recevoir un ou plusieurs dispositifs récepteurs, au moins l'un de ces dispositifs récepteurs contenant un anticorps pouvant être obtenu par un procédé suivant la revendication 5.
